# EUROPÄISCHE PATENTSCHRIFT

(11) **EP 1 722 741 B1**
(45) Veröffentlichungstag und Bekanntmachung des Hinweises auf die Patenterteilung: **01.04.2009**
(21) Anmeldenummer: 05707377.7
(22) Anmeldetag: 14.02.2005
(51) Int. Cl.: A61K 6/093, A61K 6/10

(54) **DENTALMATERIAL AUF BASIS VON ALKOXYSILYLFUNKTIONELLEN POLYETHERN**
DENTAL MATERIAL BASED ON ALKOXYSILYL-FUNCTIONAL POLYETHERS
PRODUIT DENTAIRE A BASE DE POLYETHERS A FONCTION ALCOXYSILYLE

(30) Priorität: 13.02.2004 DE 102004008022
(43) Veröffentlichungstag der Anmeldung: 22.11.2006
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 58540 Meinerzhagen (DE)
(74) Vertreter: KEIL & SCHAAFHAUSEN Patentanwälte
(86) Internationale Anmeldenummer: PCT/EP2005/001470
(87) Internationale Veröffentlichungsnummer: WO 2005/077321

(56) Entgegenhaltungen:
- EP-A- 1 081 191
- EP-A- 1 226 808
- EP-A- 1 402 873

## Beschreibung

Die vorliegende Erfindung betrifft kondensationsvemetzende Dentalmaterialien, insbesondere kondensationsvemetzende Zweikomponenten-Dentalabformmaterialien, auf Basis von alkoxysilylfunktionellen Polyethern, welche insbesondere zur Abdrucknahme geeignet sind, und deren Verwendung. Derartige Materialien werden in der Dentalmedizin bspw. zur Zahnabdrucknahme, Bissregistrierung, Zahnprothesenunterfütterung, als provisorischer und permanenter Dentalzement, provisorisches Verschlussmaterial oder dentales Zahntechnik-Dubliermaterial eingesetzt.

Bekannte kondensationsvernetzende Dentalmaterialien enthalten üblicherweise hydroxylfunktionelle Polymere mit einem Siliconrückgrat, welche in Gegenwart organischer Zinnverbindungen als Katalysatoren. Alkoxysilanen und/oder Kieselsäurestern als Vernetzer und Wasser aushärten. Allerdings sind derartige Materialien aufgrund des Siliconrückgrats der Polymere vergleichsweise hydrophob, so dass diesen zwecks Herabsetzung der Oberflächenspannung und zur Einstellung der erforderlichen Benetzbarkeit erhebliche Anteile an Tensiden zugefügt werden müssen. Ein weiterer Nachteil dieser Zusammensetzungen liegt in dem Einsatz toxikologisch bedenklicher organischer Zinnverbindungen als Katalysator.

Alternativ dazu sind Zweikomponenten-Dentalmaterialien bekannt, welche terminale Alkoxysilylgruppen aufweisende Polymere mit einem hydrophilen Polyetherrückgrat enthalten, die zur Benetzung der feuchten Zahnsubstanz ausreichend hydrophile Eigenschaften aufweisen. Üblicherweise bestehen diese Materialien aus einer Basiskomponente enthaltend alkoxysilylfunktionelle Polyether mit einem mittleren Molekulargewicht von 800 bis 20.000 g/mol, welche synthesebedingt auch Harnstoff- und/oder Urethangruppen aufweisen können, Füllstoffe sowie ggf. weitere Zusatzstoffe, und einer Katalysatorkomponente, welche neben Füll- und ggf. weiteren Hilfsstoffen eine organische und/oder anorganische Säure als Katalysator enthält.

Aus der EP 0 269 819 B1 sind kondensationsvemetzende Zweikomponenten-Dentalmaterialien bekannt, deren Basiskomponente Alkoxysilylendgruppen enthaltende Polyadditionsprodukte mit einer überwiegend linearen Molekülstruktur und einem mittleren Molekulargewicht von 800 bis 20.000 g/mol enthalten, welche einen Gehalt an Polyethergruppen von 25 bis 90 Gew.-%, einen Gehalt an Urethangruppen von 0,5 bis 10 Gew.-%, einen Gehalt an Harnstoffgruppen von 0,5 bis 10 Gew.-% sowie einen Gehalt an terminalen Alkoxysilylgruppen von 1 bis 25 Gew.-%, und, deren Katalysatorkomponente eine Abmischung enthaltend Wasser sowie organische und/oder anorganische Säuren in Gewichtsmengenverhältnissen (Wasser/Säure) von 1:0,01 bis 1:40, aufweisen. Allerdings ist die Synthese der in der Basiskomponente enthaltenden funktionellen Polyetherpolymere sehr aufwendig und teuer. Ein weiterer Nachteil dieser Dentalmaterialien liegt in dem Einsatz säurehaltiger Katalysatoren. Zum einen können durch die Säurekatalysatoren bei der Abformung im Patientenmund die Mundschleimhaut und der Zahnschmelz durch Säureätzung geschädigt werden. Zudem erlauben diese Systeme keinen Zusatz an stickstoffbasenhaltigen Substanzen, wie Adstringentien, bspw. Epinephrine, oder anderen säurelabilen therapeutischen Zusätze, da diese durch den Säurekatalysator aufgrund von Protonierung oder Spaltung inaktiviert werden. Ferner erfordert der Einsatz von Säuren bei der Produktion der Dentalmaterialien entsprechende Sicherheitsvorkehrungen.

In der EP 1 226 808 A2 werden kondensationsvernetzende Zweikomponenten-Dentalmaterialien bestehend aus einer Basis- und Katalysatorkomponente offenbart, deren Basiskomponente alkoxysilylfunktionelle Polyether mit linearer oder verzweigter Hauptkette und einem mittleren Molekulargewicht von 800 bis 20.000 g/mol enthalten, welche einen Gehalt an Polyethergruppen von 20 bis 95 Gew.-%, einen Gehalt an terminalen Alkoxysilylgruppen von 0,2 bis 25 Gew.-% sowie ggf. einen Gehalt an Urethangruppen oder Harnstoffgruppen von bis zu 10 Gew.-%, und, deren Katalysatorkomponente eine Mischung enthaltend Wasser sowie organische und/oder anorganische Säuren in Gewichtsmengenverhältnissen von 1:0,01 bis 1:40, aufweisen. Vorzugsweise enthält die Katalysatorkomponente p-Toluolsulfonsäurehydrat als Katalysator sowie ein Polyetherdiol und weitere Zusatzstoffe, wie Füllstoffe, Paraffin, Emulgator und dergl. Zwar sind die in diesen Dentalmaterialien eingesetzten funktionellen Polyetherpolymere gegenüber den zuvor genannten einfacher und kostengünstiger zu synthetisieren und zeichnen sich durch eine bessere Abbindekinetik aus. Allerdings machen auch diese Dentalmaterialien von säurehaltigen Katalysatoren Gebrauch, so dass einerseits die Gefahr der Schädigung der Mundschleimhaut sowie des Zahnschmelzes bei der Abformung im Patientenmund besteht und diesen Materialien zudem keine säurelabilen therapeutischen Zusätze zugesetzt werden können. Ein weiterer Nachteil der Systeme liegt in deren geringeren Lagerstabilität. Eine unabhängig von der Lagerungszeit gleichbleibende Abbindezeit ist allerdings eine der wichtigsten Anforderungen an ein dentales Abformmaterial.

In der WO 99/48942 wird vorgeschlagen, zur Vernetzung von als Kleb- oder Dichtstoffen einzusetzenden und Polyethergruppen aufweisenden Polyurethanen metalllorganische Verbindungen, wie Eisen- oder Zinnverbindungen, bspw. Zinn(II)oktat, oder tertiäre Amine, wie Triethylamin, einzusetzen. Ein Nachteil dieser Katalysatoren liegt jedoch in deren hohen Toxizität, so dass bei der Produktion der Materialien geeignete sicherheitstechnische Vorkehrungen zu treffen sind und eine Verwendung der Materialien als Dentalabformmassen nicht ohne weiteres möglich ist. Zudem sind insbesondere tertiäre Amine geruchsintensiv, weshalb deren Einsatz in Dentalmaterialien unerwünscht ist. Ferner sind die in diesen Materialien eingesetzten Polyurethane aufgrund deren hohen Anteil an Urethangruppen pro Molekül durch starke intermolekulare Wechselwirkungen gekennzeichnet, was bei gegebener Kettenlänge der Moleküle zu einer verglichen mit alkoxysilylfunktionellen Polyethern, die pro Molekül nur zwei Urethangruppen aufweisen, zu einer erhöhten Viskosität der Materialien führt, weswegen in diesen Materialien weniger Füllstoffe eingesetzt werden können, was wiederum erhöhte Herstellungskosten bedingt.

Aufgabe der vorliegenden Erfindung ist es daher, ein hydrophiles kondensationsvernetzendes Dentalmaterial, insbesondere ein kondensationsvernetzendes Zweikomponenten-Dentalabformmaterial, auf Basis von Alkoxysilylpolyethem zur Verfügung zu stellen, welches lagerstabil ist, insbesondere eine konstante Reaktionskinetik auch nach mindestens 18 Monaten Lagerungszeit gewährleistet, eine gute Biokompatibilität aufweist, insbesondere geruchs- und geschmackneutral ist, zudem den Zusatz säurelabiler Zusätze, wie stickstoffhaltiger Adstringentien, Medikamente, Bakterizide, Fungizide und dergl. ermöglicht und toxikologisch möglichst unbedenkliche Inhaltsstoffe aufweist.

Erfindungsgemäß wird diese Aufgabe durch ein kondensationsvernetzendes Dentalmaterial der Zusammensetzung gemäß Patentanspruch 1 gelöst.

Überraschenderweise konnte im Rahmen der vorliegenden Erfindung gefunden werden, dass die erfindungsgemäß einzusetzenden Salzkatalysatoren eine gute katalytische Aktivität für Kondensationsreaktionen aufweisen und diese daher hervorragend dafür geeignet sind, in kondensationsvernetzenden Dentalmaterialien auf Basis von alkoxysilylfunktionellen Polyethern als Katalysator eingesetzt zu werden. Die erfindungsgemäßen Dentalmaterialien weisen nicht nur eine für Dentalmaterialien geeignete Reaktionskinetik auf, sondern insbesondere auch eine praxisgerechte Verarbeitungs- und Abbindezeit. Dabei zeichnen sich diese Katalysatoren gegenüber den zu diesem Zweck bisher bekannten Substanzen, wie metallorganischen Verbindungen und tertiären Amine, durch gute Biokompatibilität aus und erfordern bei der Herstellung der Dentalmaterialien weniger rigide Sicherheitsvorkehrungen. Insbesondere kann auf den Einsatz schwermetallhaltiger Katalysatoren, wie zinn-, zink- oder bleiorganische Katalysatorverbindungen, verzichtet werden. Ein weiterer Vorteil der erfindungsgemäß eingesetzten Salzkatalysatoren insbesondere gegenüber den aus dem Stand der Technik bekannten primären, sekundären und tertiären Aminen liegt in deren Geruchs- und Geschmacksneutralität, was für ein dentales Abformmaterial eine wichtige Eigenschaft ist, um die Patientenakzeptanz zu erreichen und bspw. Würgereaktionen des Patienten bei der Applikation zu vermeiden. Zudem war es für den Fachmann unerwartet, dass derartige Dentalmaterialien auch nach mehrmonatiger Lagerung eine gleich bleibende Reaktionskinetik, insbesondere eine konstante Verarbeitungs- und Abbindezeit, aufweisen. Dies liegt u.a. darin begründet, dass die erfindungsgemäß eingesetzten Katalysatorsalze während der Lagerzeit und nach dem Aushärten keine Neben- oder Abbaureaktionen mit anderen Inhaltsstoffen, wie bspw. mit den Füllstoffen, mit dem ggf. als Pastenbildner eingesetzten Polyether oder dem Alkoxysilylpolyether, eingehen. Abgesehen davon ist der Einsatz eines Salzes als Katalysator verglichen mit dem einer freien Säure und einer freien Base, wie einem tertiärem Amin, auch deshalb vorteilhaft, weil das Salz einen moderaten pH-Wert aufweist, wodurch eine gute Verträglichkeit der erfindungsgemäßen Dentalmaterialien mit der Mundschleimhaut und dem Zahnschmelz gewährleistet wird, so dass bei der Applikation keine Verätzungen oder Irritationen auftreten. Ein weiterer Vorteil der erfindungsgemäßen Dentalmaterialien liegt darin, dass diesen auch säurelabile Zusätze, insbesondere stickstoffhaltige Adstringentien, Medikamente, Bakterizide, Fungizide und dergl., zugesetzt werden können, ohne dass diese während der Lagerung abgebaut werden.

Die erfindungsgemäßen Dentalmaterialien können sowohl als Einkomponentenals auch als Zweikomponentenmaterial formuliert sein. Während die Formulierung der Einkomponenten-Dentalmaterialien möglichst absolut wasserfrei sein muss, um eine Reaktion der alkoxysilylfunktionellen Polyether während der Lagerung zu verhindern, und die Reaktion der alkoxysilylfunktionellen Polyether nach der Applikation der Materialien auf dem abzuformenden Gegenstand durch Luftfeuchtigkeit initiiert wird, ist der Katalysatorkomponente des erfindungsgemäßen Zweikomponenten-Dentalmaterials vorzugsweise Wasser zugesetzt. Vorzugsweise werden die erfindungsgemäßen Zweikomponenten-Dentalmaterialien so formuliert, dass die

### Basiskomponente A

a) wenigstens einen alkoxysilylfunktionellen Polyether,
   und die Katalysatorkomponente B
b) wenigstens einen Katalysator und
c) Wasser
enthält, wobei der wenigstens eine Katalysator b) ein Salz ist, welches gebildet ist aus wenigstens einem aus der aus
- Komplexen von Alkalimetall- und Ammonium-Kationen mit Kronenethern und/oder Kryptanden,
- Tetraalkyl-, Tetraaryl-, Trialkylaryl-, Dialkyldiaryl-, Monoalkyltriarylammonium-Kationen, Tetraalkyl-, Tetraaryl-, Trialkylaryl-, Dialkyldiaryl-, Monoalkyltriarylphosphonium-Kationen, Tetraalkyl-, Tetraaryl-, Trialkylaryl-, Dialkyldiaryl-, Monoalkyltriarylarsonium-Kationen, Tetraalkyl-, Tetraaryl-, Trialkylaryl-, Dialkyldiaryl-, Monoalkyltriarylstibonium-Kationen,
- durch Protonierung einer Base gebildete Kationen, wobei die Base einen in Acetonitril gemessenen pK_{BH+}-Wert von mindestens 20 aufweist,
und Kombinationen hiervon bestehenden Gruppe ausgewählten Kation und wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist. Unabhängig von der Formulierung als Ein- oder Zweikomponentenmaterial ist das Anion des erfindungsgemäß einzusetzenden Katalysatorsalzes vorzugsweise ein Anion einer verzweigten Carbonsäure mit einer Länge der an der Carboxylgruppe vorgesehenen (Cyclo)Alkylkette von wenigstens 3 Kohlenstoffatomen, besonders bevorzugt wenigstens 4 und ganz besonders bevorzugt wenigstens 5 Kohlenstoffatomen oder einer unverzweigten Carbonsäure mit einer Länge der an der Carboxylgruppe vorgesehenen (Cyclo)Alkylkette von wenigstens 5 Kohlenstoffatomen, wobei insbesondere Anionen von entsprechenden (cyclo)aliphatischen Monocarbonsäuren bevorzugt sind.

Gemäß einer ersten bevorzugten Ausführungsform der vorliegenden Erfindung wird als Katalysator b) wenigstens ein Salz gebildet aus wenigstens einem Kronenether-Alkalimetallion-Komplex, Kronenether-Ammoniumion-Komplex, Kryptanden-Alkalimetallion-Komplex und/oder Kryptanden-Ammoniumion-Komplex sowie wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist, eingesetzt.

Vorzugsweise wird in dieser Ausführungsform als Kation des Katalysatorsalzes ein Komplex gebildet aus einem oder mehreren Lithium-, Natrium-, Kalium-, Rubidium-, Cäsium- und/oder Ammonium-lonen und einem oder mehreren der nachfolgenden Kronenether und/oder Kryptanden eingesetzt:
15-Crown-5, 18-Crown-6, Dibenzo-18-crown-6, Dibenzo-21-crown-7, Dibenzo-24-crown-8, Dibenzo-30-crown-10, 1,4,10-Trioxa-7,13-diazacyclopentadecan, 4,7,13,18-Tetraoxa-1,10-diazabicyclo[8.5.5]eicosan, 1,4,10,13-Tetraoxa-7,16-diazacyclooctadecan, 3,6,9,14-Tetrathiabicyclo[9.2.1]tetradeca-11,13-dien, 1,4,7,10-Tetrathiacyclododecan, 1,5,9,13-Tetrathiacyclohexadecan-3,11-diol, 1,5,9-Triazacyclododecan, 1,4,7-Triazacyclononan, 1,4,7,10,13,16-Hexamethyl-1,4,7,10,13,16-hexaazacyclooctadecan,

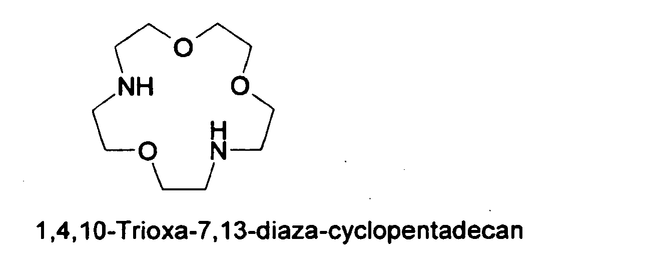

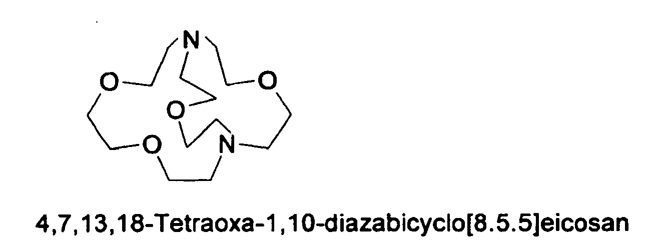

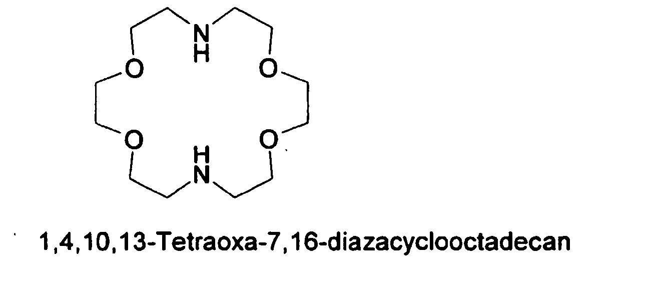

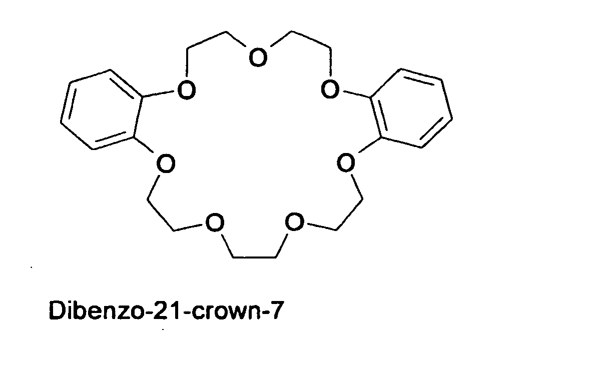

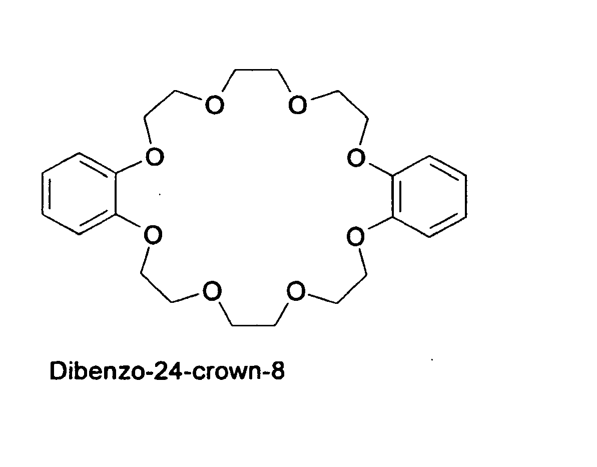

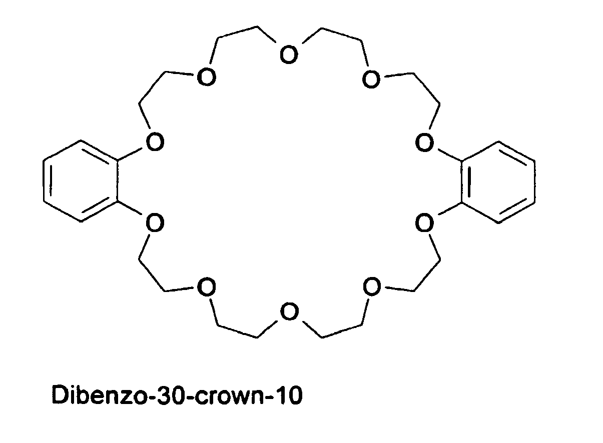

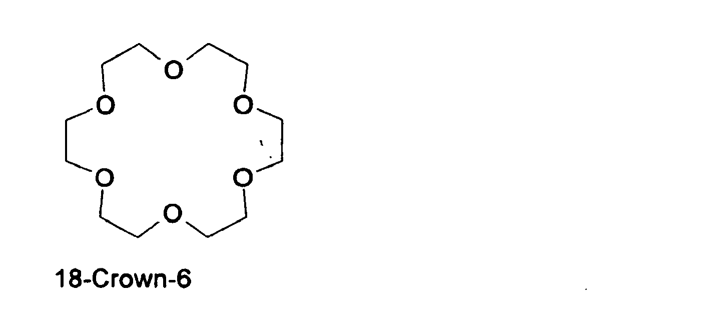

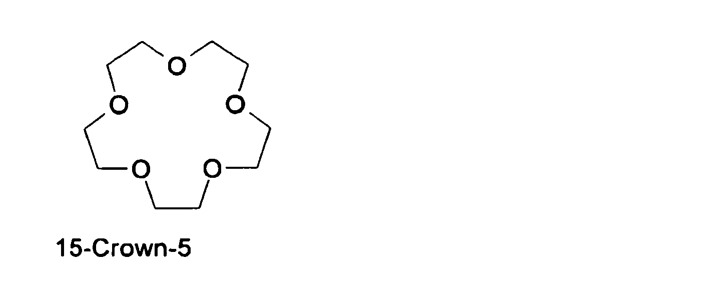

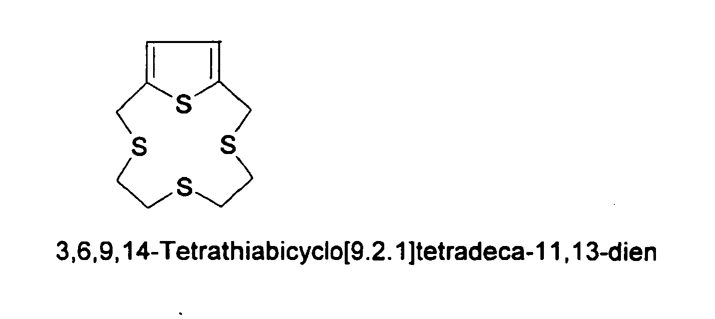

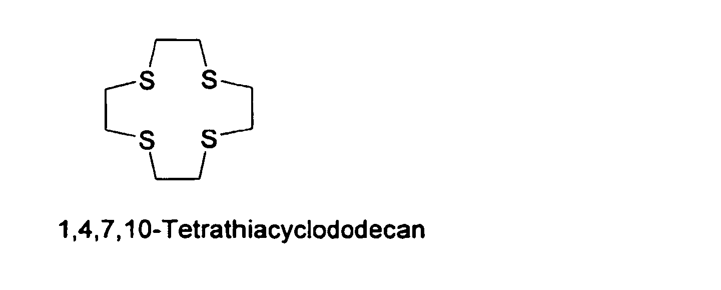

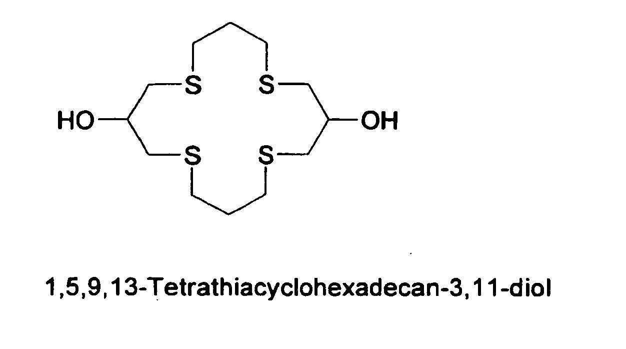

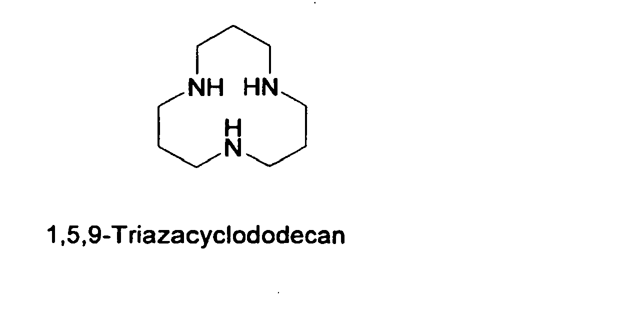

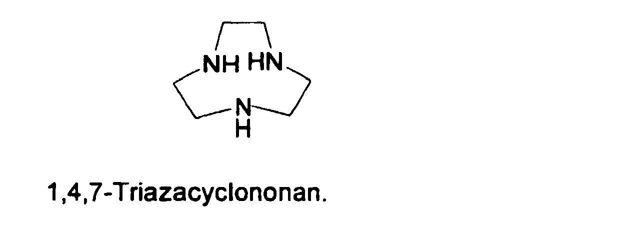

Die Herstellung des in dieser Ausführungsform erfindungsgemäß einzusetzenden Katalysatorsalzes kann auf jede dem Fachmann bekannte Art erfolgen, beispielsweise durch folgende Reaktion:

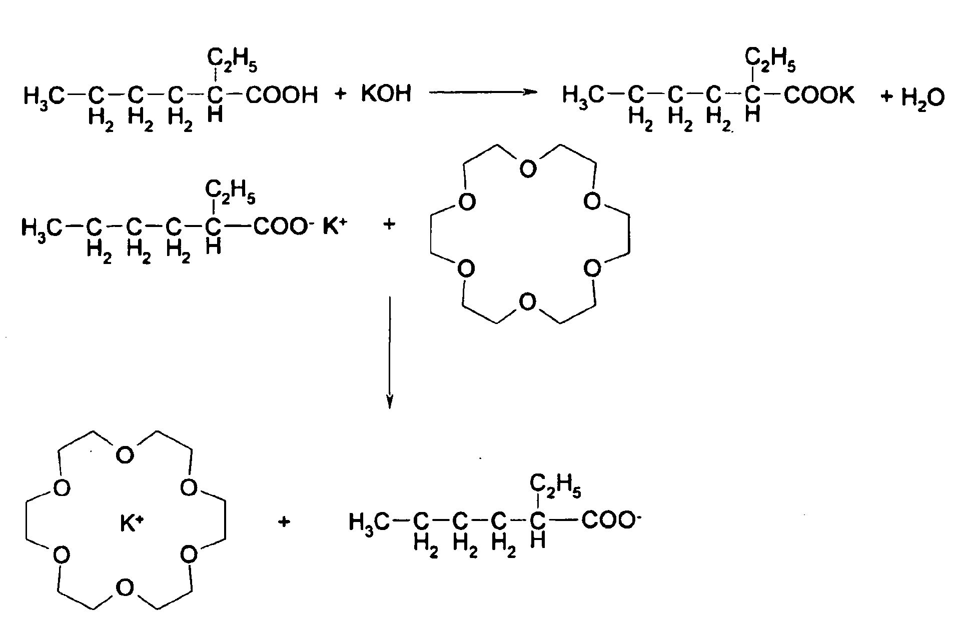

Gemäß einer zweiten bevorzugten Ausführungsform der vorliegenden Erfindung wird als Katalysator b) wenigstens ein Salz gebildet aus wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist, und einem Tetraalkyl-, Tetraaryl-, Trialkylaryl-, Dialkyldiaryl-, Monoalkyltriarylammonium-, Tetraalkyl-, Tetraaryl-, Trialkylaryl-, Dialkyldiaryl-, Monoalkyltriarylphosphonium-, Tetraalkyl-, Tetraaryl-, Trialkylaryl-, Dialkyldiaryl-, Monoalkyltriarylarsonium-, Tetraalkyl-, Tetraaryl-, Trialkylaryl-, Dialkyldiaryl-, und/oder Monoalkyltriarylstibonium-Kation eingesetzt. Beispiele für geeignete Kationen für die gemäß dieser Ausführungsform einzusetzenden Katalysatorsalze sind Tetramethylammonium-, Tetraethylammonium-, Tetrapropylammonium-, Tetrabutylammonium-, Tetrapentylammonium-, Tetrahexylammonium-, Tetraheptylammonium-, Tetraoctylammonium-, Tetranonylammonium-, Tetradecylammonium-, Tetramethylphosphonium-, Tetraethylphosphonium-, Tetrapropylphosphonium-, Tetrabutylphosphonium-, Tetrapentylphosphonium-, Tetrahexylphosphonium-, Tetraheptylphosphonium-, Tetraoctylphosphonium-, Tetranonylphosphonium-, Tetradecylphosphonium-, Tetramethylarsonium-, Tetraethylarsonium-, Tetrapropylarsonium-, Tetrabutylarsonium-, Tetrapentylarsonium-, Tetrahexylarsonium-, Tetraheptylarsonium-, Tetraoctylarsonium-, Tetranonylarsonium-, Tetradecylarsonium-, Tetramethylstibonium-, Tetraethylstibonium-, Tetrapropylstibonium-, Tetrabutylstibonium-, Tetrapentylstibonium-, Tetrahexylstibonium-, Tetraheptylstibonium-, Tetraoctylstibonium-, Tetranonylstibonium-, Tetradecylstibonium-, Lauryltrimethylammonium-, Myristyltrimethylammonium-, Cetyltrimethylammonium-, Stearyltrimethylammonium-, Lauralkonium-, Myristalkonium-, Cetalkonium-, Stearalkonium-, Cetylethyldimethylammonium-, Benzyltriethylammonium- und Benzalkonium-lonen.

Die Herstellung der Salze aus den vorgenannten Carbonsäureanionen und den alkyl- und/oder arylgruppensubstituierten Ammonium-/Phosphonium-/ Arsonium-/Stiboniumionen gemäß dieser Ausführungsform kann auf jede dem Fachmann bekannte Weise erfolgen, wobei sich beispielsweise die folgender Synthese als besonders geeignet erwiesen hat:

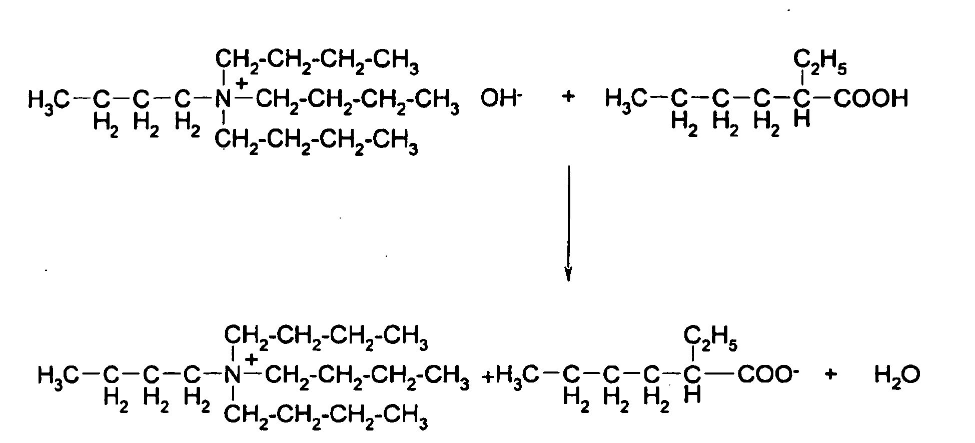

Gemäß einer dritten bevorzugten Ausführungsform der vorliegenden Erfindung wird als Katalysator b) wenigstens ein Salz gebildet aus wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist und einem durch Protonierung einer Base mit einem in Acetonitril gemessenen pK_{BH+}-Wert von mindestens 20 gebildeten Kation eingesetzt. Besonders gute Ergebnisse werden erhalten, wenn das Dentalmaterial als Katalysator b) ein aus einem vorgenannten Anion und einem durch Protonierung einer Base mit einem in Acetonitril gemessenen pK_{BH+}-Wert von mindestens 21, besonders bevorzugt mindestens 22 und ganz besonders bevorzugt mindestens 23 gebildeten Kation gebildetes Salz enthält.

In Weiterbildung des Erfindungsgedankens wird vorgeschlagen, in der dritten Ausführungsform der vorliegenden Erfindung als Katalysator b) ein aus einem Anion einer der vorgenannten Carbonsäuren und einer protonierten Base gebildetes Salz einzusetzen, wobei die Base eine Struktur aufweist, welche nach Protonierung der Base eine Mesomeriestabilisierung der positiven Ladung ermöglicht. Mesomeriestabilisierung im Sinne der vorliegenden Erfindung bedeutet im Einklang mit dem allgemeinen Lehrbuchwissen, dass sich für die protonierte Base wenigstens zwei Grenzstrukturen formulieren lassen, in denen die positive Ladung an unterschiedlichen Atomen lokalisiert ist, bzw., dass in der protonierten Base π-Elektronen delokalisiert sind. Bevorzugt sind insbesondere aus einem Anion einer der vorgenannten Carbonsäuren und einer protonierten Base gebildete Katalysatorsalze, wobei die Base wenigstens eine Struktureinheit gemäß der allgemeinen Formel I 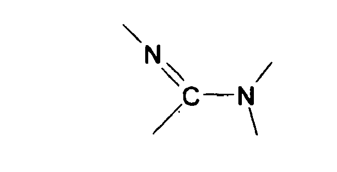 und/oder gemäß der allgemeinen Formel II 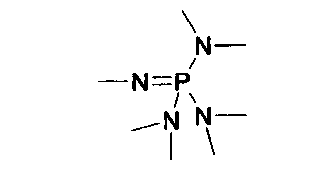 und/oder gemäß der allgemeinen Formel III 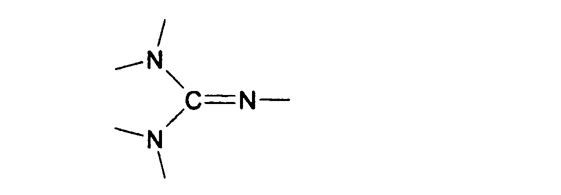 aufweist. Diese Struktureinheiten führen nach der Protonierung der Base zu einer guten Mesomeriestabilisierung der positiven Ladung, was zu einer Stabilisierung der protonierten Form führt.

Insbesondere werden für die dritte Ausführungsform der vorliegenden Erfindung gute Ergebnisse erzielt, wenn als Kation eine aus der aus 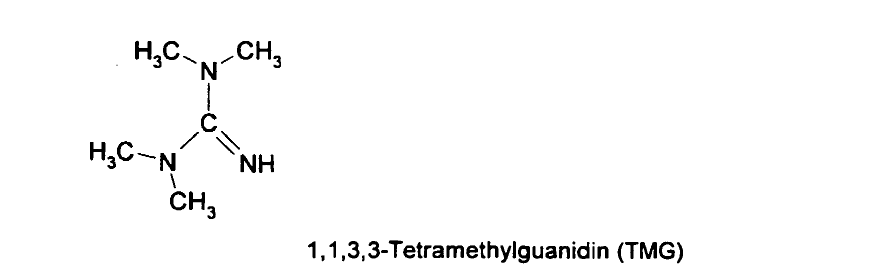 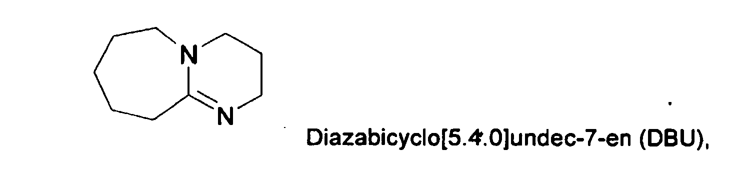 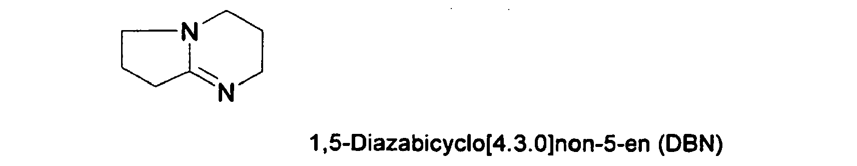 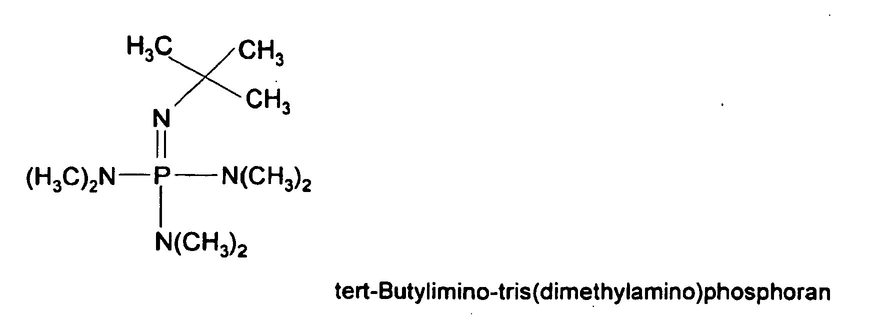 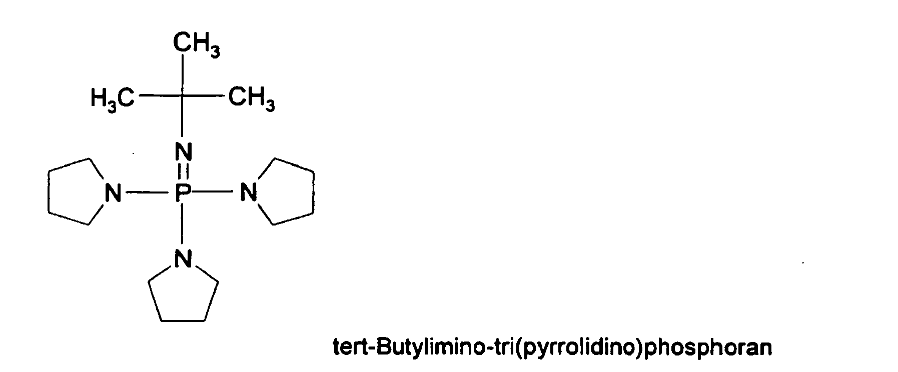 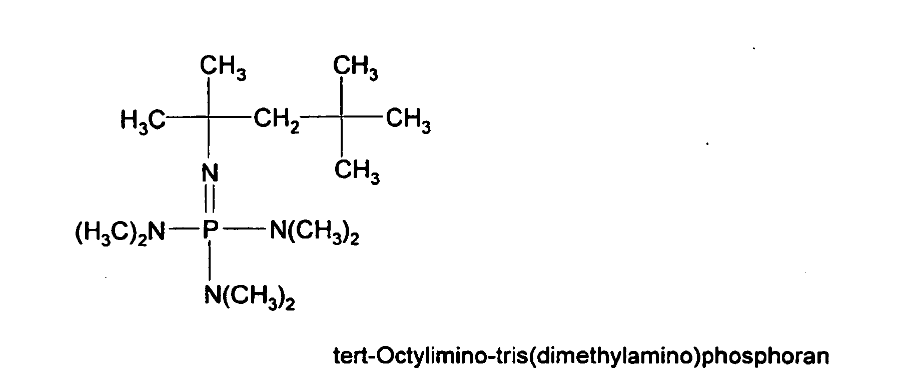 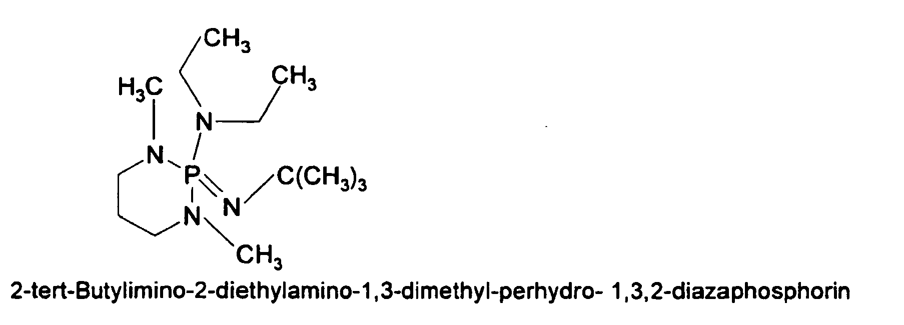 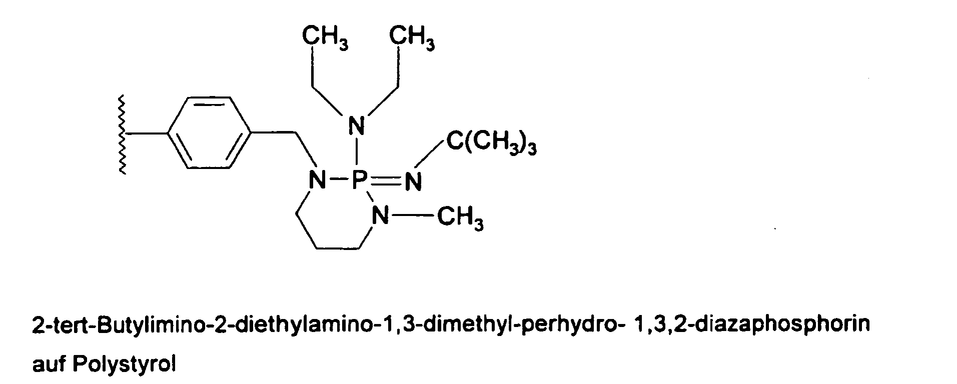 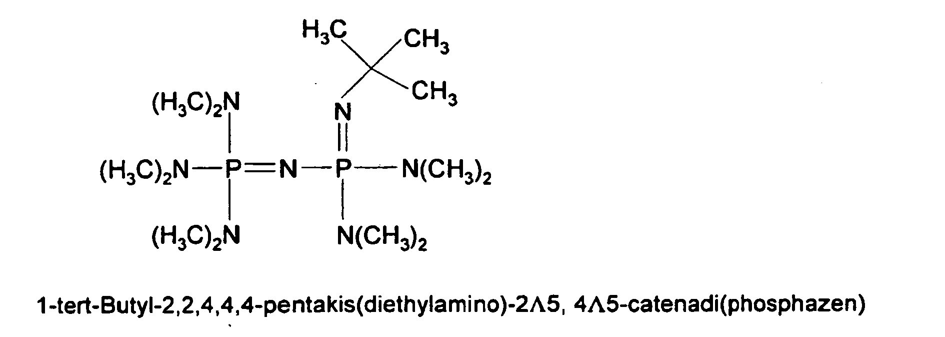 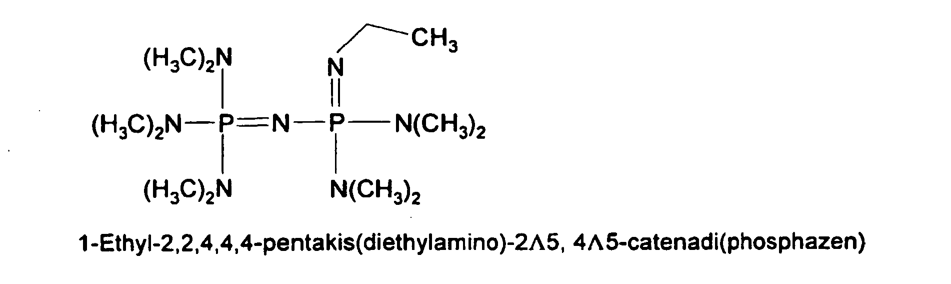 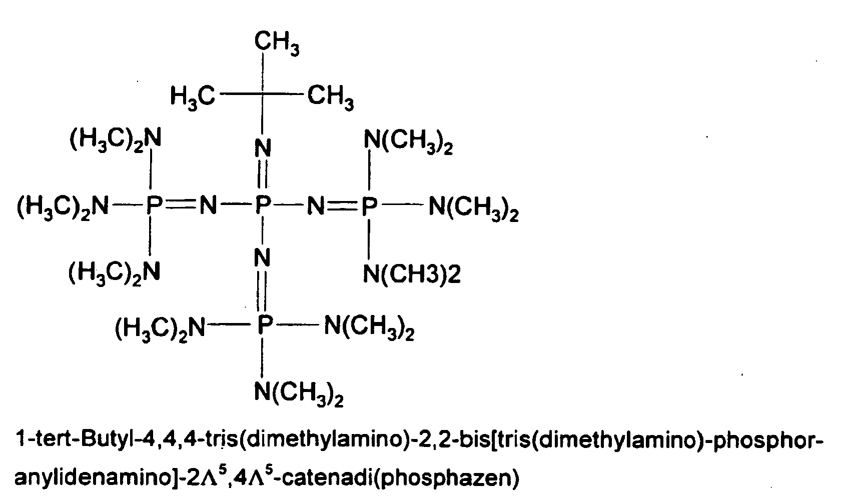 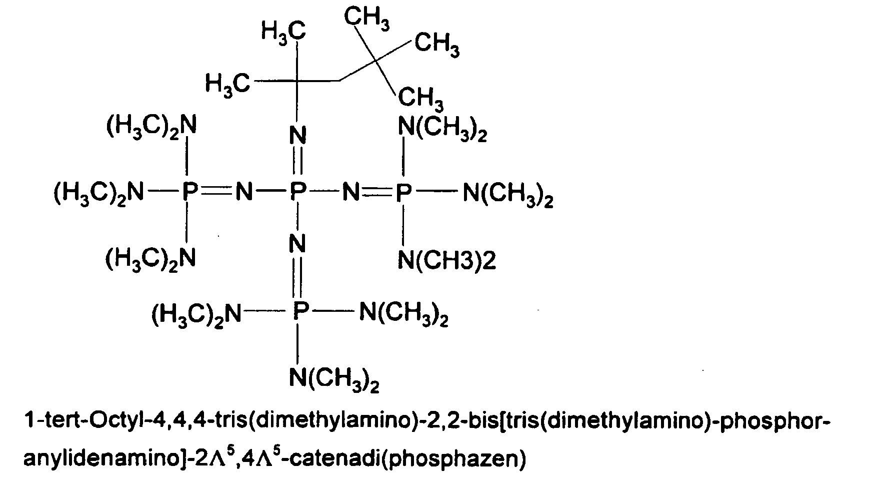 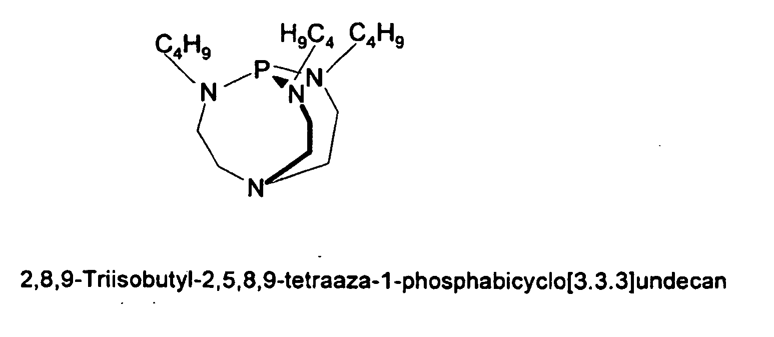 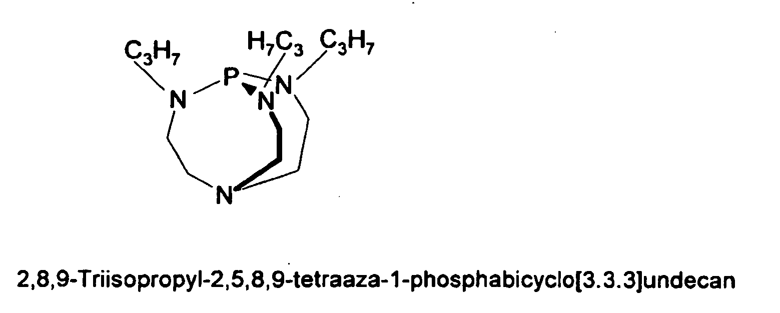 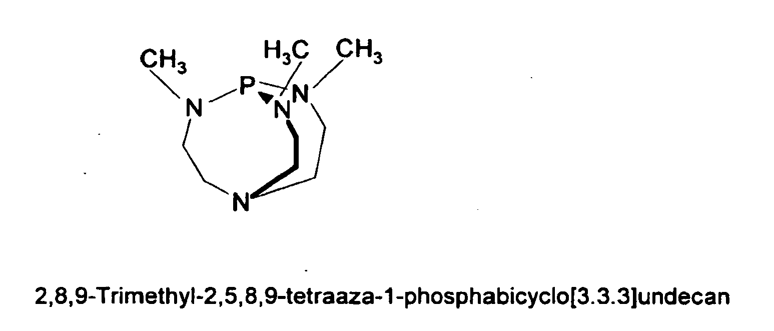 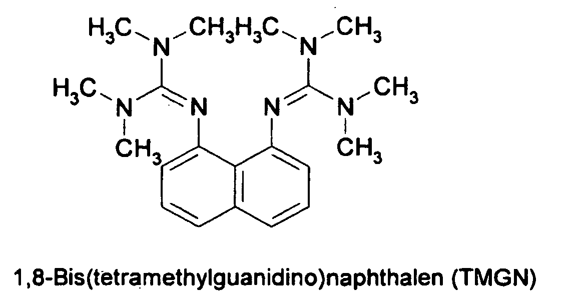 sowie 2-tert-Butyl-1,1,3,3-tetramethylguanidin, 1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählte, protonierte Base eingesetzt wird.

Ganz besonders bevorzugt ist die Basenkomponente des Salzes gemäß der dritten Ausführungsform der vorliegenden Erfindung tert-Butyliminotri(pyrrolidino)phosphoran, 1-tert-Butyl-2,2,4,4,4-pentakis(diethylamino)-2A5, 4Λ5-catenadi(phosphazen), 1-tert-Butyl-4,4,4-tris(dimethylamino)-2,2-bis[tris(dimethylamino)-phosphoranylidenamino]-2Λ⁵,4Λ⁵-catenadi(phosphazen), tert-Octylimino-tris(dimethylamino)phosphoran, 2,8,9-Tri-isopropyl-2,5,8,9-tetraaza-1-phosphabicyclo[3.3.3]undecan, 1,5-Diazabicyclo[4.3.0]non-5-en, 1,1,3,3-Tetramethylguanidin, Diazabicyclo[5.4.0]undec-7-en, 7-Methyl-1,5,7-triazabicyclo(4.4.0)dec-5-en, 2-tert-Butyl-1,1,3,3-tetra-methylguanidin, 1,5,7-Triazabicyclo(4.4.0)dec-5-en und/oder 1,8-Bis(tetramethylguanidino)naphthalen.

Die Herstellung des durch Säure-Base-Reaktion gebildeten Katalysatorsalzes gemäß der dritten Ausführungsform der vorliegenden Erfindung kann auf jede dem Fachmann bekannte Weise erfolgen, beispielsweise durch die nachfolgende Reaktion:

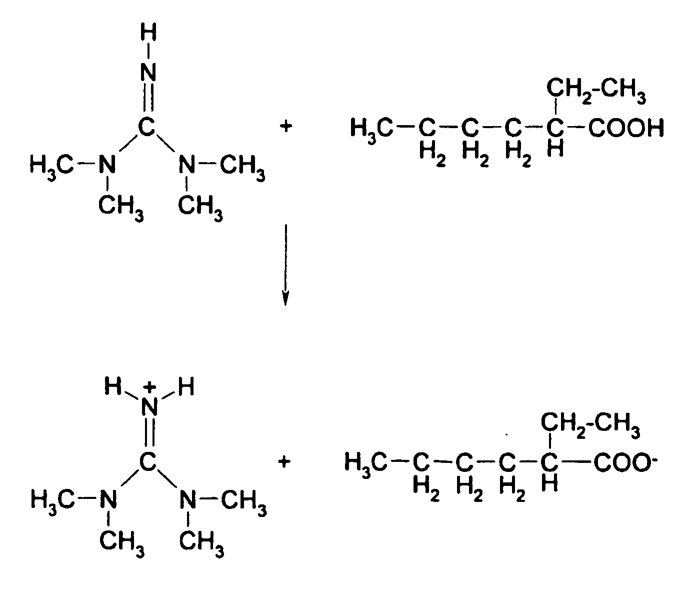

Erfindungsgemäß können in den Dentalmaterialien als Katalysator alle aus wenigstens einem der vorgenannten Kationen und wenigstens einem der vorgenannten Carbonsäureanionen gebildeten Salze eingesetzt werden, wobei insbesondere Salze, in denen das Anion des Katalysatorsalzes ein Anion einer verzweigten Carbonsäure mit einer Länge der an der Carboxylgruppe vorgesehenen (Cyclo)Alkylkette von wenigstens 3 Kohlenstoffatomen, besonders bevorzugt wenigstens 4 und ganz besonders bevorzugt wenigstens 5 Kohlenstoffatomen oder einer unverzweigten Carbonsäure mit einer Länge der an der Carboxylgruppe vorgesehenen (Cyclo)Alkylkette von wenigstens 5 Kohlenstoffatomen ist, bevorzugt sind. Des weiteren bevorzugt sind Salze, in denen das Anion ein Anion einer gesättigten und/oder ungesättigten (cyclo)aliphatischen Monocarbonsäure mit einer der zuvor genannten (Cyclo)Alkylkettenlänge ist.

Besonders gute Ergebnisse werden insbesondere auch mit Salzen umfassend wenigstens ein durch Deprotonierung resultierendes aliphatisches oder cycloaliphatisches Carbonsäureanion mit einer wenigstens eine und bevorzugt wenigstens zwei Verzweigungen aufweisenden (Cyclo)Alkylkette erzielt. Vorzugsweise wird als Anion des wenigstens einen Katalysatorsalzes ein aliphatisches oder cycloaliphatisches Carbonsäureanion eingesetzt, das wenigstens eine Verzweigung in γ-Stellung, besonders bevorzugt wenigstens eine Verzweigung β-Stellung und ganz besonders bevorzugt wenigstens eine Verzweigung in α-Stellung zur Carboxylgruppe des Carbonsäureanions aufweist. Ebenso bevorzugt sind entsprechende Carbonsäureanionen, in deren (Cyclo)Alkylkette sowohl in γ-, β- und/oder α-Stellung zu der Carboxylgruppe des Carbonsäureanions jeweils wenigstens eine Verzweigung vorgesehen ist.

In Weiterbildung des Erfindungsgedankens wird insbesondere für die dritte Ausführungsform, in der das Kation des Katalysatorsalzes durch Protonierung einer Base gebildet wird, vorgeschlagen, in dem Dentalmaterial als Katalysator b) wenigstens ein Salz gebildet aus einer der vorgenannten Kationen und einem Anion einer aus der aus 2,2-Dialkylalkansäure, 3,3-Dialkylalkansäure, 4,4-Dialkylalkansäure, 2,3-Dialkylalkansäure, 2,4-Dialkylalkansäure, 3,4-Dialkylalkansäure, 2,2-Dialkylalkensäure, 3,3-Dialkylalkensäure, 4,4-Dialkylalkensäure, 2,3-Dialkylalkensäure, 2,4-Dialkylalkensäure, 3,4-Dialkylalkensäure, 2,2-Dialkylalkinsäure, 3,3-Dialkylalkinsäure, 4,4-Dialkylalkinsäure, 2,3-Dialkylalkinsäure, 2,4-Dialkylalkinsäure, 3,4-Dialkylalkinsäure, 2-Monoalkylalkansäure, 3-Monoalkylalkansäure, 4-Monoalkylalkansäure, 2,2-Dialkylhexansäure, bevorzugt 2,2-Dialkylnonan-säure, 2,2-Dimethyldecansäure, 2,2-Diethyldecansäure, 2,2-Dipropyldecan-säure, 2,2-Dibutyldecansäure, 2,2-Dimethylnonansäure, 2,2 Diethylnonansäure, 2,2-Dipropylnonansäure, 2,2-Dibutylnonansäure, 2,2-Dimethyloctansäure, 2,2-Diethyloctansäure, 2,2-Dipropyloctansäure, 2,2-Dibutyloctansäure, 2,2-Dimethylheptansäure, 2,2-Diethylheptansäure, 2,2-Dipropylheptansäure, 2,2-Dibutylheptansäure, 2,2-Dimethylhexansäure, 2,2-Diethylhexansäure, 2,2-Dipropylhexansäure, 2,2-Dibutylhexansäure, 2-Butyloctansäure, 2-Hexyldecansäure, 2-Propylpentan-säure, 1-Methyl-1-cyclohexancarbonsäure, 2,2-Dimethylbuttersäure, 2,2-Dimethylvaleriansäure, 3,5,5-Trimethylhexansäure, 2-Ethylhexansäure, Decansäure, Octansäure, Hexansäure und Önanthsäure bestehenden Gruppe ausgewählten Säure vorzusehen.

Gemäß einer weiteren besonderen Ausführungsform der vorliegenden Erfindung ist das Dentalmaterial als Zweikomponentensystem formuliert und enthält als Katalysator b) wenigstens ein Salz von Diazabicyclo[5.4.0]undec-7-en, Diazabicyclo[4.3.0]non-5-en und/oder 1,1,3,3-Tetramethylguanidin mit 2-Alkylalkansäure, insbesondere 2-Alkylhexansäure, 2-Ethylhexansäure, 2,2-Dialkylalkansäure, 2,2-Dialkylhexansäure, 2,2-Dialkylnonansäure, 2,2-Dimethylhexansäure, 2,2-Diethylhexansäure, 2,2-Dimethylnonansäure, 2,2-Diethylnonansäure und/oder 2-Propylpentansäure.

In Weiterbildung des Erfindungsgedankens wird vorgeschlagen, dass die in den erfindungsgemäß einzusetzenden Katalysatorsalzen vorgesehenen Kationen und/oder Carbonsäureanionen Alkoxysilylgruppen aufweisen. Dadurch wird erreicht, dass das Katalysatorsalz nach Aushärten des Dentalmaterials in der Polyethermatrix eingebunden ist und aus dem dentalen Abformmaterial nicht mehr herausgelöst werden kann.

Erfindungsgemäß können die Dentalmaterialien als Katalysator b) eines oder, in beliebiger Kombination miteinander, mehrere der zuvor genannten Salze enthalten. Vorzugsweise enthält das Dentalmaterial nur eines der zuvor genannten Salze als Katalysator, wobei besonders bevorzugt neben dem einen oder mehreren erfindungsgemäß einzusetzenden Salzen keine weiteren Katalysatoren, insbesondere keine metallorganischen Metallsalze, tertiären Amine oder freie Säuren, eingesetzt werden.

Vorzugsweise wird in dem erfindungsgemäßen Dentalmaterial ein Katalysatorsalz b) mit einem in Wasser (Ampuwa, pH 5,8) gemessenen pH-Wert zwischen 7 und 11 und besonders bevorzugt zwischen 7 und 9 eingesetzt.

Wie der Fachmann erkennt hängt die Menge an einzusetzendem Katalysatorsalz u.a. von der Löslichkeit des Salzes in der eingesetzten Polyethermatrix ab. Vorzugsweise beträgt die Menge an einzusetzendem Katalysatorsalz, bezogen auf die Gesamtmischung des Dentalmaterials, 0,001 bis 1 mmol/g, besonders bevorzugt 0,001 bis 0,5 mmol/g, ganz besonders bevorzugt 0,001 bis 0,1 mmol/g und höchst bevorzugt 0,005 bis 0,05 mmol/g. Selbstverständlich muss das eingesetzte Katalysatorsalz eine Mindestlöslichkeit in der eingesetzten Polyethermatrix aufweisen, um überhaupt katalytisch wirken zu können.

Um die Menge an einzusetzendem Katalysatorsalz möglichst gering zu halten, wird in Weiterbildung des Erfindungsgedankens vorgeschlagen, in dem Dentalmaterial ein Katalysatorsalz mit einer hinreichend hohen Löslichkeit in dem Polyethermaterial, d.h. mit einer ausreichenden katalytischen Aktivität, einzusetzen, wobei die katalytische Aktivität im Sinne der vorliegenden Patentanmeldung durch die Aushärtezeit nach ISO 4823 (Ausgabe 1992), bestimmt durch Rückstellung nach Verformung, charakterisiert ist. Vorzugsweise wird ein Katalysatorsalz eingesetzt, dass mit Polytetrahydrofuran, Polyethylenglycol und besonders bevorzugt Polypropylenglycol sowie deren Mischungen und Copolymerisate als Polyethermatrix eine Aushärtezeit von kleiner gleich 30 min, besonders bevorzugt kleiner gleich 15 min für eine Zahntechnik-Dubliermasse bzw. eine Aushärtezeit von kleiner gleich 15 min, besonders bevorzugt kleiner gleich 10 min, ganz besonders bevorzugt kleiner gleich 7 min und höchst bevorzugt kleiner gleich 6 min für eine dentale Abformmasse ergibt.

Wenn das erfindungsgemäße Dentalmaterial als Einkomponentenmaterial formuliert ist, sollte es möglichst absolut wasserfrei sein, um eine Reaktion der alkoxysilylfunktionellen Polyether während der Lagerung zu vermeiden.

In dem Fall, dass das Dentalmaterial als Zweikomponentenmaterial formuliert ist, enthält die Katalysatorkomponente B vorzugsweise Wasser, wohingegen die Basiskomponente A möglichst absolut wasserfrei ist. Vorzugsweise enthält die Katalysatorkomponente B des erfindungsgemäßen Zweikomponenten-Dentalmaterials, bezogen auf die Gesamtmischung, 0,005 bis 3 mmol/g, besonders bevorzugt 0,01 bis 2 mmol/g und ganz besonders bevorzugt 0,02 bis 1 mmol/g an Wasser.

Vorzugsweise enthält das erfindungsgemäße Dentalmaterials wenigstens einen verstärkenden Füllstoff d₁) und/oder wenigstens einen nicht-verstärkenden Füllstoff d₂). Bei der Formulierung als Zweikomponenten-Material kann die Basiskomponente A wenigstens einen der vorgenannten Füllstoffe enthalten, wobei vorzugsweise sowohl in der Basiskomponente A als auch in der Katalysatorkomponente B wenigstens ein verstärkender Füllstoff und/oder wenigstens ein nicht-verstärkender Füllstoff vorgesehen ist.

Als verstärkende Füllstoffe d₁) eignen sich insbesondere hochdisperse, aktive Füllstoffe mit einer BET-Oberfläche von wenigstens 50 m²/g und/oder einer Primärpartikelgröße von kleiner gleich 100 nm, besonders bevorzugt kleiner gleich 80 nm. Besonders geeignet sind solche mit einer Primärpartikelgröße im Nanometerbereich, welche als Aggregate und/oder Agglomerate vorliegen können. Bevorzugt ist der wenigstens eine verstärkende Füllstoffe d₁) eine Substanz ausgewählt aus der Gruppe, welche aus Aluminiumhydroxid, Zinkoxid, Titandioxid, Zirkoniumoxid, Siliciumdioxid sowie gefällter und/oder pyrogener Kieselsäure besteht. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff d₁) in Form von Nanopartikeln, als faser- oder blättchenförmiger Füllstoff, bspw. mineralischer, faserförmiger Füllstoff, oder als synthetischer, faserförmiger Füllstoff vor.

In Weiterbildung des Erfindungsgedankens wird vorgeschlagen, bei Formulierung als Zweikomponentenmaterialien vorzugsweise in der Basiskomponente A, verstärkende Füllstoffe d₁) vorzusehen, die einen Wassergehalt von maximal 0,5 Gew.-%, besonders bevorzugt von maximal 0,3 Gew.-%, ganz besonders bevorzugt von maximal 0,15 Gew.-% aufweisen und höchst bevorzugt absolut bzw. im Wesentlichen wasserfrei sind, wobei der Wassergehalt über Karl-Fischer Titration bestimmt wird.

Gemäß einer weiteren besonderen Ausführungsform der vorliegenden Erfindung, weist der wenigstens eine verstärkende Füllstoff d₁) mit einer BET-Oberfläche von größer 50 m²/g in der Basiskomponente A einen pH-Wert von 5 bis 11, bevorzugt von 5 bis 9 und besonders bevorzugt von 5,5 bis 8,5, auf. Auf diese Weise wird eine Degradierung der alkoxysilylfunktionellen Polyether während der Lagerung vermieden.

Bei Formulierung als Zweikomponentensystem enthält vorzugsweise die Basiskomponente A, bezogen auf die Komponente A, 0 bis 50 Gew.-%, besonders bevorzugt 0,1 bis 40 Gew.-% und ganz besonders bevorzugt 0,1 bis 30 Gew.-%, und die Katalysatorkomponente B, bezogen auf die Komponente B, 0 bis 50 Gew.-%, besonders bevorzugt 0,1 bis 40 Gew.-% und ganz besonders bevorzugt 0,1 bis 30 Gew.-% wenigstens eines verstärkenden Füllstoffs d₁).

Als nichtverstärkende Füllstoffe d₂) eignen sich prinzipiell dieselben Substanzen wie für die verstärkende Füllstoffe d₁), wobei die nichtverstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugt ist der wenigstens eine nichtverstärkende Füllstoffe d₂) eine Substanz ausgewählt aus der Gruppe, welche aus Erdalkalimetalloxiden, Erdalkalimetallhydroxiden, Erdalkalimetallfluorid, Erdalkalimetallcarbonaten, Calciumapatit (Ca₅[(F, Cl, OH, ½CO₃) | (PO₄)₃], insbesondere Calciumhydroxylapatit (Ca₅[(OH) | (PO₄)₃]), Titandioxid, Zirkoniumoxid, Aluminiumhydroxid, Siliciumdioxid, gefällter Kieselsäure und Calciumcarbonat besteht. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe d₂) eine mittlere Korngröße von größer als 0,1 µm (Ullmann Encyclopädie der Technischen Chemie, Band 21, Seite 523) auf.

In Weiterbildung des Erfindungsgedankens wird bei Formulierung des Dentalmaterials als Zweikomponentensystem vorgeschlagen, dass der wenigstens eine nichtverstärkende Füllstoff d₂) in der Basiskomponente A einen Wassergehalt von maximal 0,5 Gew.-%, besonders bevorzugt maximal 0,1 Gew.-%, ganz besonders bevorzugt maximal 0,05 Gew.-% aufweist und höchst bevorzugt absolut bzw. im Wesentlichen wasserfrei ist.

Gemäß einer besonderen Ausführungsform der vorliegenden Erfindung weist der wenigstens eine nichtverstärkende Füllstoff d₂) in der Basiskomponente A einen pH-Wert von 5 bis 11, bevorzugt von 5 bis 9 und besonders bevorzugt von 5,5 bis 8,5, auf, um eine Degradierung der alkoxysilylfunktionellen Polyether während der Lagerung zu vermeiden.

Vorzugsweise enthält die Basiskomponente A des erfindungsgemäßen Dentalmaterials, bezogen auf die Komponente A, 0 bis 80 Gew.-%, besonders bevorzugt 0,05 bis 75 Gew.-% und ganz besonders bevorzugt 0,1 bis 70 Gew.-%, und die Katalysatorkomponente B, bezogen auf die Komponente B, 0 bis 80 Gew.-%, besonders bevorzugt 0,05 bis 75 Gew.-% und ganz besonders bevorzugt 0,1 bis 70 Gew.-% wenigstens eines nichtverstärkenden Füllstoffs d₂).

In Weiterbildung des Erfindungsgedankens wird vorgeschlagen, dass bei Formulierung als Zweikomponentenmaterial die in der Katalysatorkomponente B enthaltenen verstärkenden und/oder nichtverstärkenden Füllstoffe einen pH-Wert zwischen 6,0 und 11,0 und ganz besonders bevorzugt solche mit einem pH-Wert zwischen 7,0 und 10,0 aufweisen.

Insgesamt beträgt der Gesamtgehalt an Füllstoffen sowohl bei der Formulierung des Dentalmaterials als Ein- als auch als Zweikomponentensystem, bezogen auf die Gesamtmischung, 0 bis 80 Gew.-%, bevorzugt 0,01 bis 80 Gew.-%, besonders bevorzugt 0,1 bis 75 Gew.-% und ganz besonders bevorzugt 0,2 bis 70 Gew.-%.

Als alkoxysilylfunktionelle Polyether a) können prinzipiell alle Polyether enthaltend Alkoxysilylgruppen eingesetzt werden, wobei das Polyetherrückgrat linear und/oder verzweigt und beispielsweise aus Polyethylenoxid, Polypropylenoxid, Polytetrahydrofuran und/oder deren Copolymeren aufgebaut sein kann, wobei diese Monomere statistisch, blockweise oder in einer taktischen Ordnung vorliegen können. Als Starter für die Polyether und/oder Copolymere können ein-oder mehrwertige Alkohole verwendet werden, wie bspw. Methanol, Butanol, Glycerin, Trimethylpropan, Pentaerythrit und Sorbitol. Beispielsweise können Copolymere aus Polytetrahydrofuran mit Polyethylenoxid oder aus Polyethylenoxid und Polypropylenoxid eingesetzt werden, wobei reines Polypropylenoxid besonders bevorzugt ist. Ferner bevorzugt sind Polyether mit seitenständigen Alkylgruppen, wobei jede oder wenigstens jede zehnte Monomerstruktureinheit eine seitenständige Alkylgruppe trägt. Geeignete Handelsprodukte sind Acclaim 4200, Acclaim 6320N, Acclaim 12200, Acclaim 8200 und Acclaim 6300 der Bayer AG, Polyglycol P41/300 und Polyglycol P41/3000 (Clariant) sowie Poly-(ethylenglycol-ran-propylenglycol) (Aldrich). Bevorzugt weisen die Polyether a) ein zahlengemitteltes Molekulargewicht von 500 bis 25.000 g/mol und besonders bevorzugt von 5.000 bis 20.000 g/mol auf.

Ein Auswahlkriterium für den erfindungsgemäß geeigneten Polyether ist neben der Kettenlänge (Elastizität), der Alkoxysilylfunktionalisierung (Aushärte-Kinetik) und der Anzahl der Urethan-/Harnstoffgruppen (Viskosität, Rheologie) die Hydrophilie des Polyethers, die über die Anzahl, Struktur und Polarität der Monomerwiederholungseinheiten des Polyetherpolymers bestimmt wird. Die Hydrophilie für ein Dentalabformmaterial muss einerseits ausreichend hoch sein, um ein gutes Anfließen an feuchte Zahnsubstanz sicherzustellen (niedrige Kontaktwinkel), aber andererseits darf das Material nicht zu hydrophil sein, da sonst Wasser, Feuchtigkeit oder Speichel während der Abdrucknahme bzw. beim Desinfizieren bzw. beim Ausgießen mit Gips zur Quellung führt und damit die erforderliche Dimensionsgenauigkeit nicht mehr gegeben ist. Die Hydrophilie des Polyethers ist darüber hinaus auch unter anderem für die Löslichkeit des erfindungsgemäßen Katalysators mit verantwortlich.

Gemäß einer besonderen Ausführungsform der vorliegenden Erfindung weist der wenigstens eine alkoxysilylfunktionelle Polyether einen Gehalt an Polyethergruppen zwischen 5 und 30 mmol/g und besonders bevorzugt zwischen 10 und 25 mmol/g auf.

Vorzugsweise ist die Alkoxysilylstruktureinheit bzw. sind die Alkoxysilylstruktureinheiten des Polyethers a), bezogen auf das Polymerrückgrat, terminal angeordnet und fallen unter die allgemeine Formel IV

- SiR⁵R⁶R⁷

worin R⁵, R⁶ und R⁷ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

In Weiterbildung des Erfindungsgedankens wird vorgeschlagen, dass der wenigstens eine Polyether a) einen Alkoxygruppengehalt von 0,02 bis 12 mmol/g, besonders bevorzugt von 0,04 bis 6 und ganz besonders bevorzugt von 0,04 bis 3 mmol/g, aufweist.

Durch die Art und Anzahl der Alkoxygruppen pro Siliziumatom lässt sich die Kondensationskinetik und damit der Verarbeitungs- und Abbindezeit des Dentalmaterials einstellen. Bevorzugt werden diese Parameter derart gewählt, dass die Verarbeitungszeit 30 Sekunden bis 3 Minuten, besonders bevorzugt zwischen 1 und 2,5 Minuten und ganz besonders bevorzugt zwischen 1,5 und 2 Minuten und/oder die nach ISO 4823 (Ausgabe 1992) bestimmte Abbindezeit im Patientenmund (sog. Mundverweildauer) maximal 15 Minuten, besonders bevorzugt maximal 10 Minuten, ganz besonders bevorzugt maximal 7 Minuten und höchst bevorzugt 6 Minuten beträgt.

Vorzugsweise weist der wenigstens eine Polyether a) als dritte Struktureinheit (neben den terminalen Alkoxygruppen und den Polyethergruppen) jeweils an den terminalen Alkoxysilylgruppen angeordnete Alkylenspacer, welche bevorzugt C₁-C₆-Alkylgruppen, besonders bevorzugt C₁-C₃-Alkylgruppen, ganz besonders bevorzugt Ethylengruppen und/oder Methylengruppen und höchst bevorzugt Methylengruppen sind, auf.

Zudem kann der wenigstens eine Polyether a) als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweisen. Insbesondere dann, wenn der wenigstens eine Polyether a) als vierte Struktureinheit Harnstoff- und/oder Urethangruppen aufweist, ist eine Methylengruppe als Spacer bevorzugt. Durch den Einsatz solcher α-aktivierter Alkoxysilylpolyether werden hydrophile, lagerstabile Zweikomponenten-Dentalabformmassen erhalten, welche mit einem erfindungsgemäß als Katalysator einzusetzenden Salz überraschend schnell durch Kondensationsreaktion vernetzen. Insgesamt sollte der Gehalt an Urethan- bzw. Harnstoffgruppen pro Molekül so gering wie möglich gehalten werden, um die intermolekularen Wechselwirkungen zwischen den einzelnen Polyetherketten zu minimieren, um die durch den Polyetherzusatz begründete Viskosität so gering wie möglich zu halten, was den Zusatz höherer Mengen an Füllstoffen in dem Dentalmaterial ermöglicht und somit mehr Formulierungsfreiräume und kostengünstigere Rezepturen einräumt.

Im Rahmen der vorliegenden Erfindung hat es sich als vorteilhaft erwiesen, Polyether, die innerhalb der Polyetherkette keine Urethan- oder Harnstoffgruppen enthalten und die an jedem Kettenende höchstens/nicht mehr als eine Urethan-oder Harnstoffgruppe und höchstens/nicht mehr als eine (Alkoxy)silylgruppe und höchstens/nicht mehr als eine Methylenspacergruppe tragen, einzusetzen. Der Einsatz dieser Polyether führt verglichen mit den im Stand der Technik eingesetzten Polyurethan-Alkoxysilylpolyethern zu Formulierungen mit niedrigerer Viskosität, so dass den Dentalmaterialien mehr Füllstoffe zugegeben werden können, was zu einer Reduzierung der Herstellkosten führt.

Gemäß einer weiteren besonderen Ausführungsform der vorliegenden Erfindung sind die einzelnen Struktureinheiten des wenigstens einen Polyethers a) folgendermaßen angeordnet: 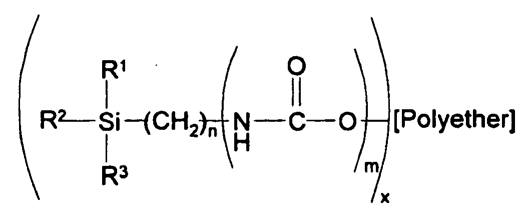 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl-oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist.
oder 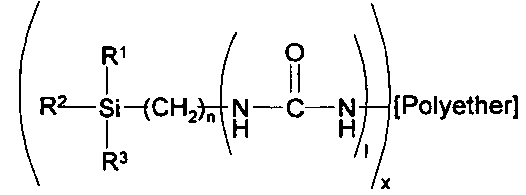 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl-oder Methylgruppen, besonders bevorzugt Ethoxy-, Methoxy-, Ethyl-oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie I=0 oder 1, besonders bevorzugt I=1 ist.

Gemäß einer besonderen Ausführung der vorliegenden Erfindung ist der Alkylspacer in den vorgenannten Struktureinheiten Methylen (n=1).

Die Herstellung dieser alkoxysilylfunktionellen Polyether ist bekannt und wird beispielsweise in der DE 101 04 079 A1, EP 0 629 819 B1, DE 101 39 132, US 4,906,707, EP 0 372 561 A1, EP 1 303 560 A1 und EP 0 170 865 B1, welche hiermit als Referenz eingeführt und als Teil der Offenbarung gelten, beschrieben. Beispiele für kommerziell erhältliche und im Rahmen der vorliegenden Erfindung geeignete Polyether sind MS Polymer S 203H, MS Polymer S 303H (Kaneka), Polymer XP ST55, ST50, ST51, ST53 (Hanse), SLM 414000 (Wacker), SLM 414001 (Wacker), Baycoll XP 2458 und Desmoseal XP 2447 (Bayer AG), wobei der unter dem Handelsnamen SLM 414000 vertriebene Dimethoxy(methyl)silylmethylcarbamat-terminierte Polyether : 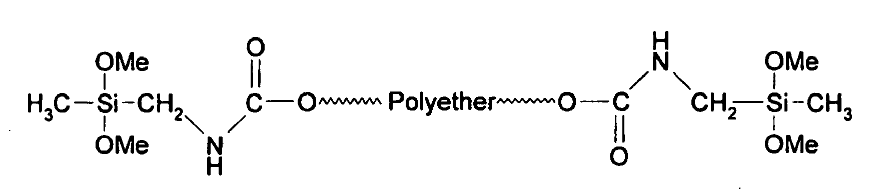 und Dimethoxy(methyl)silylmethylharnstoff-terminierte Polyether: 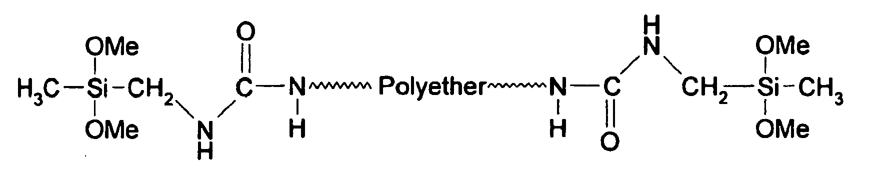 besonders bevorzugt sind.

In Weiterbildung des Erfindungsgedankens wird vorgeschlagen, dem erfindungsgemäßen Dentalmaterial ein oder mehrere der folgenden Zusatz- bzw. Hilfsstoffe zuzusetzen:
f) Thixotropierungsmittel,
g) Wasserfänger,
h) Pastenbildner,
i) Tensid,
j) Wirkstoff,
k) Weichmacher,
l) optische Abtastung ermöglichende Substanz,
m) Geschmacks- und/oder Geruchsstoff,
n) Diagnostik ermöglichende Substanz,
o) Fluoridisierungsmittel,
p) Bleichsubstanz,
q) Desensibilisierungsmittel,
r) Haftverbundvermittler,
s) Farbstoff,
t) Indikator,
u) Stabilisator (Antioxidanz, Radikalfänger).

Dem erfindungsgemäßen Dentalmaterial können optional Thixotropierungsmittel f) zugesetzt werden, wobei sich zu diesem Zweck insbesondere hochmolekulare Polyether, wie Polyethylenglykol, Polyethylenglykol-Polypropylenglykol-Copolymere, Poly-Tetrahydrofuran, Kohlenwasserstoffwachse, Amidwachse, Triglyceride, Kieselsäuren und Silicate als besonders geeignet erwiesen haben.

Gemäß einer weiteren besonderen Ausführungsform der vorliegenden Erfindung weisen die Dentalmaterialien, wenn als Zweikomponentensystem formuliert vorzugsweise in der Basiskomponente A, wenigstens einen Wasserfänger g) auf, welcher besonders bevorzugt aus der Gruppe, welche aus Alkoxysilanen, Titanaten, wie Tetraisopropyltitanat, Zirkonaten, wie Tetrapropylzirkonat, Zeolithen, Aluminiumsulfat, wasserfreiem Calciumsulfat (bspw. Drierite^{®}), Blaugel und/oder Oxazolidinen besteht, ausgewählt ist.

In Weiterbildung des Erfindungsgedankens wird vorgeschlagen, als Wasserfänger g) ein oder mehrere funktionelle Alkoxysilane einzusetzen, da durch solche Verbindungen zusätzlich die Vernetzungsgeschwindigkeit, die Struktur und die Eigenschaften des resultierenden Elastomers eingestellt werden können. Bevorzugt ist das wenigstens eine funktionelle Alkoxysilan eine Verbindung der allgemeinen Formel V

R⁸₄₋ₓ-Si-R⁹ₓ

mit R⁸=H, Alkyl, Alkenyl, -(CH₂)ₙ-Z, wobei n=1 bis 6,
R⁹=Alkoxy,
Z=NH₂, NHR, NR₂, wobei R=Alkyl, Aminoalkyl, -C(O)OCH₃, sowie
x=1, 2, 3 oder 4,
wobei besonders bevorzugt R⁸= Alkenyl, insbesondere Vinyl, oder -(CH₂)ₙ-Z mit Z=NHR und n=1 oder 3, insbesondere n=1, und/oder
x=3 und/oder R= -C(O)OCH₃.

Besonders bevorzugt ist das wenigstens eine funktionelle Alkoxysilan g) Vinyltrimethoxysilan, N-Trimethoxysilylmethyl-O-methylcarbamat und/oder eine Verbindung der folgenden Formel: 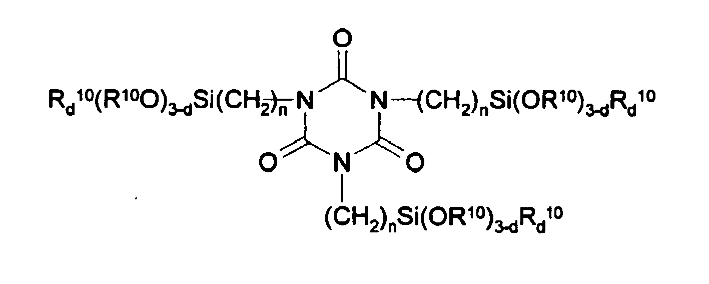 worin n = 1 bis 6, bevorzugt n = 1 oder 3, besonders bevorzugt n = 1,
d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind.

Die zuvor genannten Verbindungen sind reaktive Silane, die als Wasserfänger zur Beseitigung von in der Komponente A der Dentalzusammensetzung noch vorhandenen Wasserspuren fungieren.

Des weiteren enthalten die erfindungsgemäßen Zweikomponenten-Dentalmaterialien vorzugsweise, und zwar wenn als Zweikomponentensystem formuliert besonders bevorzugt in der Katalysatorkomponente B, wenigstens einen Pastenbildner h), da dieser die Einstellung einer pastenartigen Konsistenz, bspw. dünn-, mittel- oder zähfließend, sowie eine homogene Vermischung des Salzes und der festen verstärkenden und nichtverstärkenden Füllstoffen ermöglicht. Vorzugsweise wird als wenigstens ein Pastenbildner h) eine Verbindung ausgewählt aus der Gruppe, welche aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden, Glycerin und Poly(meth)acrylsäuren besteht, eingesetzt. Selbstverständlich können die erfindungsgemäßen Dentalmaterialien auch eine beliebige Kombination zweier oder mehrerer der zuvor genannten Verbindungen enthalten.

Besonders bevorzugt sind hydrophile Pastenbildner, in denen sich die erfindungsgemäße Katalysatorbase mit Wasser homogen mischen lässt. Die Mischbarkeit kann durch Zusatz von Tensiden noch verbessert werden. Besonders bevorzugte Vertreter sind Polyether, Polyurethane, Polyester, mehrwertige Alkohole, insbesondere Propylenglycole, Polypropylenglycole, Ethylenglycole, Polyethylenglycole, Butylenglycole, Polybutylenglycole und Glycerin, sowie Mischungen und Copolymere hiervon.

Bei den ggf. als Tensid, Emulgator und/oder Stabilisator eingesetzten Verbindungen i) handelt es sich vorzugsweise um anionische Tenside, besonders bevorzugt Alkylsulfate, Alkylbenzolsulfonate oder Alkylbenzolphosphate, kationische Tenside, besonders bevorzugt Tetraalkylammoniumhalogenide, nichtionische Tenside, besonders bevorzugt Alkyl- und Alkylphenyl-Polyalkylalkylenoxide, Fettsäurealkoxylate, Fettalkoholalkyloxylate sowie deren Alkylether und Alkylester, Fettsäurealkylolamide, Saccharosefettsäureester, Trialkylaminoxide, Silicontenside (z.B. Silwet L77, Tegopren 5878) oder Fluortenside, oder um amphotere Tenside, besonders bevorzugt sulfatierte oder oxyethylierte Kondensationsprodukte aus Alkenylphenolen und Formaldehyd, Ethylenoxid-propylenoxid-Blockpolymerisate oder modifizierte Polysiloxane. Mit Vorteil können auch in den alkoxysilylfunktionellen Polyether a) einpolymerisierbare Tenside, wie sie in der US 4,160,778, die hiermit als Referenz eingeführt und als Teil der Offenbarung gilt, offenbart sind, eingesetzt werden. Zusätzlich oder alternativ dazu können auch Derivate der zuvor genannten Tenside eingesetzt werden, bspw. solche, die über funktionelle Gruppen, wie -OH, -CH=CH₂, - OCO-(CH₃)C=CH₂ sowie Alkoxysilylgruppen, verfügen. Zudem können, wenn auch weniger bevorzugt, andere, dem Fachmann bekannte Tenside eingesetzt werden.

Bevorzugt wird als eingesetzten Verbindungen i) ein Silicontensid eingesetzt, da sich im Rahmen der vorliegenden Erfindung gezeigt hat, dass mit diesen Verbindungen überraschenderweise in der Polyethermatrix sehr niedrige, mit der Methode "liegender Tropfen" bestimmte, Kontaktwinkel erzielt werden können.

Diese Mischungen zeichnen sich durch eine ausgezeichnete Benetzbarkeit und ein hervorragendes Anfließverhalten an feuchte Zahn- und Gewebssubstanz aus. Trotz dieser guten hydrophilen Eigenschaften quillt das Material nicht bei Kontakt mit wässrigen Medien, wie Wasser, Speichel, Blut, Desinfektionsbad oder wässrigem Gipsbrei. Die gute initiale Benetzbarkeit der Mischungen ist wichtig für die detailgetreue Abformung des Abformmaterials in dem Patientenmund während der Verarbeitung und dem ersten Kontakt mit feuchter Mund-/Zahnsubstanz und drückt sich durch einen niedrigen Kontaktwinkel von kleiner 50°, insbesondere kleiner gleich 45°, gemessen mit einem Kontaktwinkelmessgerät der Firma Krüss bei 20 °C mit der Messmethode "liegender Tropfen", aus. Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 60°, insbesondere kleiner gleich 55°, aus.

Zudem können die erfindungsgemäßen Dentalmaterialien eine oder mehrere Wirkstoffe j) enthalten, welche in Abhängigkeit von deren chemischen Funktionalität bei Formulierung als Zweikomponentensystem in der Basiskomponente A oder der Katalysatorkomponente B enthalten sein können. Zu den erfindungsgemäß einzusetzenden Wirkstoffen zählen insbesondere Adstringentien, wie Epinephrine, antibakteriell und/oder antifugal wirkende Substanzen, wie Hexitidine (bspw. 5-Amino-1, 3-bis(2-ethylhexyl)-5-methylhexahydropyrimidin), Triclosane (bspw. 2,4,4'-Trichloro-2-hydroxydiphenylether) und Chlorhexidin: 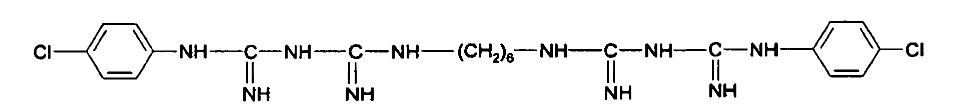 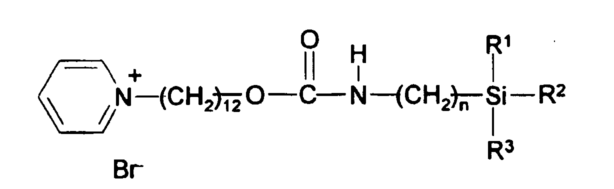 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

Als Weichmacher k) kommen insbesondere nicht reaktive Polyether, Polyester, Polyurethane, Phthalate, Mono-, Di-, Tri- oder höherwertige Ester, insbesondere Acetyltributylcitrat, Mesamoll^{®} (Bayer) und Triglyceride in Betracht, welche bei Formulierung als Zweikomponentensystem je nach deren chemischen Natur der Komponente A und/oder der Komponente B zugesetzt werden.

Als die optische Lesbarkeit/Abtastung ermöglichenden Verbindungen I) können alle dem Fachmann zu diesem Zweck bekannte Substanzen, insbesondere Metallpulver, Metallpigmente, Metallicpigmente, Zinkoxid, Zirkonoxid und Titandioxid, eingesetzt werden, welche bei Formulierung als Zweikomponentensystem je nach deren chemischen Natur der Komponente A und/oder der Komponente B zugesetzt werden.

Zudem können die erfindungsgemäßen Dentalmaterialien in einer der beiden oder in beiden Komponenten übliche Geschmacks- und/oder Geruchsstoffe m) und/oder für die Diagnostik nützliche Zusätze n) enthalten, wie sie bspw. in der EP 1 339 373, PCT/EP00/05418 und DE 100 61 195 beschrieben sind.

Als Fluoridierungshilfsstoffe o) haben sich insbesondere Natriumfluorid, Kaliumfluorid, Ammoniumfluorid, Fluorphosphate und Aminfluoride, wie N'-Octadecylbimethylendiamin-N, N, N'-bis(2-ethanol)-dihydrofluorid (wie in ZM 93, Nummer 15, Seite 32 ff. beschrieben), als geeignet erwiesen, welche bei Formulierung als Zweikomponentensystem ebenfalls in Abhängigkeit von deren chemischen Natur der Komponente A und/oder der Komponente B zugesetzt sein können.

Zudem kann das erfindungsgemäße Dentalmaterial, bei Formulierung als Zweikomponentensystem in der Komponente A und/oder der Komponente B, als Bleichsubstanz p) eine oder mehrere verschiedene Peroxide enthalten, welche vorzugsweise aus der Gruppe, welche aus Alkalimetall- und Erdalkalimetallperoxiden, Wasserstoffperoxid sowie Carbamidperoxid besteht, ausgewählt sind.

Beispiele für geeignete Desensibilisierungsmittel q) sind Kaliumsalze, wie Kaliumnitrat, Nelkenöl und Eugenol.

Als Haftverbundvermittler r), bspw. zur Ausbildung eines Haftverbundes zwischen dem Abformmaterial und einem Abformlöffel aus Edelstahl und/oder Kunststoff, eignen sich insbesondere Alkoxysilane, Epoxysilane, Aminosilane und Methacrylatsilane.

Beispiele für geeignete Farbstoffe s) sind Farbstoffpigmente in Form von Al-, Ca-, Ba-Oxiden/verlackter Farbstoff, welche wie die zuvor beschriebenen Hilfsstoffe, sofern nicht anders angegeben, bei Formulierung als Zweikomponentensystem in Abhängigkeit von deren chemischen Natur der Komponente A und/oder der Komponente B zugesetzt sein können.

Des weiteren können den erfindungsgemäßen Dentalmaterial bei Formulierung als Zweikomponentensystem in der Komponente A und/oder der Komponente B Farbstoffindikatoren t) zugesetzt sein, welche ihre Farbe in Abhängigkeit von dem pH-Wert, bspw. aufgrund von pH-Wert-Änderungen beim Vermischen der Komponenten A und B, oder beim Kontakt mit Wasser ändern.

Als Stabilisatoren und/oder Antioxidantien u) können den erfindungsgemäßen Zweikomponenten-Dentalmaterialien insbesondere aus der aus polymerem Trimethyl-dihydrochinolin, Diphenylderivaten, Phenothiazin, Phenyl-αnaphthylamin, 4, 4'-Methylen-bis-2,6-di-tert.-butylphenol, Butylhydroxytoluol, Butylhydroxyanisol (BHA) und Methoxyphenol (Hydroxyanisol) bestehenden Gruppe ausgewählte Verbindungen eingesetzt werden. Beispiele für derartige Verbindungen sind die von der Firma Ciba-Geigy kommerziell erhältlichen Produkte Irganox 1010, 1076, 1035, MD 1024, Irgafos 168, 38, Irgacor 252 LD/252FC, 1405, 1930, 153, Tinuvin 328, P, 384, 900, 928, 327, 1130, 400, 292, 144, 123, 622 sowie Chimassorb 119.

Vorzugsweise wird das erfindungsgemäße Zweikomponenten-Dentalmaterial in geeigneten Primärverpackungen, wie Tuben, Dosen, und besonders bevorzugt in Kartuschen und Schlauchbeuteln, wie sie bspw. in der EP 0 723 807 A2, EP-A-0 541 972, PCT/EP/980193, EP-A-0 492 412, EP-A-0 492 413 und EP 0 956 908 A1, welche hiermit als Referenz eingeführt und somit als Teil der Offenbarung gelten, beschrieben sind, gelagert und auf die spätere Verwendung zugeschnitten proportioniert ist.

Ein weiterer Gegenstand der vorliegenden Erfindung sind Mischungen, welche durch Vermischen der Komponenten A und B des zuvor beschriebenen erfindungsgemäßen Zweikomponenten-Dentalmaterials erhältlich sind. Vorzugsweise wird die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1 oder 5:1, vermischt.

Im Folgenden wird die Erfindung anhand von den Erfindungsgedanken demonstrierenden, diesen aber nicht einschränkenden Beispielen erläutert.

### Beispiele 1 bis 12 (erfindungsgemäß)

### (Herstellung verschiedener Katalysatorkomponenten B mit Katalysatorsalzen gebildet aus 2-Ethylhexansäure und verschiedenen starken Basen gemäß der dritten Ausführungsform der vorliegenden Erfindung)

### Herstellung verschiedener Katalysatorkomponenten B

Verschiedene Katalysatorsalze gebildet aus den in der Tabelle 1 angegebenen Mengen starker Basen und 2-Ethylhexansäure wurden in 5,0 Teilen entmineralisiertem Wasser (Ampuwa, pH 5,8) gelöst. Anschließend wurden die einzelnen Salzlösungen in einem Vakuummischbecher mit 36 Teilen Polypropylenglykol mit einer Molmasse von 4.000 g/mol, 51 Teilen Aluminiumhydroxid mit einer mittleren Korngröße von 13 µm und einer BET-Oberfläche von kleiner 1 m²/g und 5 Teilen Kieselsäure mit einer BET-Oberfläche von 130 m²/g für 5 Minuten gemischt. Danach wurde weitere 30 Minuten unter Vakuum homogen gemischt.

Es wurden mittelfließende Materialien (ISO 4823) erhalten, die verschiedene Katalysatorkomponenten B des erfindungsgemäßen Abformmaterials auf Basis von Alkoxysilylpolyethem darstellen. Die Materialien wurden in Schlauchbeutel (Verbundfolie PE/AI/PE) abgefüllt und aufbewahrt.

### Herstellung einer Basiskomponente A

In einem Vakuummischer wurden unter trockener Argon-Schutzgasatmosphäre 39 Teile eines Polypropylenglykols, das endständig über Urethangruppen und Methylenspacer mit Dimethoxymethylsilylgruppen funktionalisiert war, wobei das funktionalisierte Polypropylenglykol eine Viskosität bei 20°C von 10.000 mPa·s aufwies, mit 51 Teilen eines getrockneten mit Trimethylsilylgruppen oberflächenmodifizierten Cristobalitfüllstoffs mit einer mittleren Korngröße von 7 µm, 7 Teilen einer getrockneten, hochdispersen, pyrogen hergestellten, hydrophobierten Kieselsäure mit einer BET-Oberfläche von 170 m²/g, 0,4 Teilen Vinyltrimethoxysilan, 0,4 Teilen N-Trimethoxysilylmethyl-O-methylcarbamat und 2 Teilen Silicontensid für 5 Minuten gemischt. Danach wurde weitere 30 Minuten unter Vakuum homogen gemischt.

Es wurden mittelfließende Materialien (ISO 4823) erhalten, welche die Basiskomponente A des erfindungsgemäßen Abformmaterials auf Basis von Alkoxysilylpolyethern darstellen. Die Materialien wurden in Schlauchbeutel (Verbundfolie PE/AI/PE) abgefüllt und aufbewahrt.

### Mischung der Katalysatorkomponenten B und der Basiskomponente A

Jeweils 1 Teil der zuvor beschriebenen Katalysatorkomponenten B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Dentalmaterialien auf Basis von Alkoxysilylpolyethern sind in der Tabelle 1 sowie weitere anwendungstechnische Eigenschaften für das in Beispiel 9 erhaltene Dentalmaterial in den Tabellen 3, 4 und 5 zusammengefasst.

Die Beispiele 1 bis 12 zeigen, dass die erfindungsgemäßen Zusammensetzungen enthaltend als Katalysator ein Salz gebildet aus einer starken Basen mit einem in Acetonitril gemessenen pK_{BH+}-Wert von mehr als 20 und 2-Ethylhexansäure überraschenderweise eine für kondensationsvemetzende Alkoxysilylpolyether-Systeme hervorragende Aushärtekinetik aufweisen. Es lassen sich schnellabbindende Abformmaterialien mit praxisgerechten Verarbeitungszeiten herstellen. Der Fachmann erkennt, dass die Abbindezeit der einzelnen Beispiele durch Erhöhung bzw. Reduzierung der Menge an eingesetzten Katalysatorsalz einfach auf einen gewünschten Wert eingestellt werden kann.

Wie insbesondere aus der Tabelle 5 für die in Beispiel 9 erhaltene Masse hervorgeht erfüllen die erfindungsgemäßen Dentalmaterialien alle Anforderungen für ein funktionsfähiges dentales Abformmaterial, vor allem im Hinblick auf Shore A-Härten, Rückstellung nach der Verformung, Konsistenzen der Einzelkomponenten und der Mischung, lineare Maßänderungen und Kontaktwinkel. Insbesondere werden für die durch Kontaktwinkelmessung bestimmte Hydrophilie hervorragende Werte erhalten.

### Vergleichsbeispiele 1 bis 7 (nicht erfindungsgemäß)

Verschiedene Salze gebildet aus den in der Tabelle 1 angegebenen Mengen schwacher Basen und 2-Ethylhexansäure wurden in 5,0 Teilen entmineralisiertem Wasser (Ampuwa, pH 5,8) gelöst und gleichermaßen wie in den Beispielen 1 bis 12 zu einer Katalysatorkomponente B verarbeitet.

Jeweils 1 Teil der zuvor beschriebenen Katalysatorkomponenten B und 5 Teile der Basiskomponente A gemäß Beispiel 1 wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Materialien auf Basis von Alkoxysilylpolyethem sind in der Tabelle 1 zusammengefasst.

Wie aus der Tabelle 1 ersichtlich weisen die in den Vergleichsbeispielen 1 bis 7 hergestellten Materialien, in denen als Katalysator jeweils ein aus 2-Ethylhexansäure und einer Base mit einem in Acetonitril gemessenen pK_{BH+}-Wert von weniger als 20 gebildetes Salz als Katalysator verwendet wurde, im Unterschied zu den erfindungsgemäßen Formulierungen inakzeptable Abbindezeiten von mehr als 60 Minuten auf bzw. reagieren nicht.

### Beispiele 13 bis 40 (erfindungsgemäß)

### (Herstellung verschiedener Katalysatorkomponenten B mit erfindungsgemäßen Katalysatorsalzen gebildet aus verschiedenen starken Basen und verschiedenen Säuren)

### Herstellung verschiedener Katalysatorkomponenten B

Verschiedene Katalysatorsalze gebildet aus den in der Tabelle 2 angegebenen Mengen starker Basen und gesättigter und ungesättigter (cyclo)aliphatischer Carbonsäuren wurden in 5,0 Teilen entmineralisiertem Wasser (Ampuwa, pH 5,8) gelöst. Anschließend wurden die einzelnen Salzlösungen in einem Vakuummischbecher mit 36 Teilen Polypropylenglycol mit einer Molmasse von 4.000 g/mol, 51 Teilen Aluminiumhydroxid mit einer mittleren Korngröße von 13 µm und einer BET-Oberfläche von kleiner 1 m²/g und 5 Teilen Kieselsäure mit einer BET-Oberfläche von 130 m²/g für 5 Minuten gemischt. Danach wurde weitere 30 Minuten unter Vakuum homogen gemischt.

Es wurden mittelfließende Materialien (ISO 4823) erhalten, die verschiedene Katalysatorkomponenten B des erfindungsgemäßen Abformmaterials auf Basis von Alkoxysilylpolyethem darstellen. Die Materialien wurden in Schlauchbeutel (Verbundfolie PE/AI/PE) abgefüllt und aufbewahrt.

### Mischung der Katalysatorkomponenten B aus und der Basiskomponente A aus Beispiel 1

Jeweils 1 Teil der zuvor beschriebenen Katalysatorkomponenten B und 5 Teile der gemäß Beispiel 1 hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Dentalmaterialien auf Basis von Alkoxysilylpolyethem sind in der Tabelle 2 zusammengefasst.

Die Beispiele 13 bis 40 zeigen, dass die erfindungsgemäßen Zusammensetzungen enthaltend als Katalysator ein aus einem Kation von Basen mit einem in Acetonitril gemessenen pK_{BH+}-Wert von mehr als 20 und einem Anion gebildet durch Deprotonierung einer gesättigten Carbonsäure mit einer Länge der an der Carboxylgruppe vorgesehenen Alkylkette von wenigstens 2 Kohlenstoffatomen oder einer unverzweigten Carbonsäure mit einer Länge der an der Carboxylgruppe vorgesehenen Alkylkette von wenigstens 4 Kohlenstoffatomen, bevorzugt einer verzweigten Carbonsäure mit einer Länge der an der Carboxylgruppe vorgesehenen (Cyclo)Alkylkette von wenigstens 3 Kohlenstoffatomen oder einer unverzweigten Carbonsäure mit einer Länge der an der Carboxylgruppe vorgesehenen (Cyclo)Alkylkette von wenigstens 5 Kohlenstoffatomen zusammengesetztes Salz als Katalysator überraschenderweise eine für kondensationsvemetzende Systeme hervorragende Aushärtekinetik aufweisen. Es lassen sich schnellabbindende Abformmaterialien mit praxisgerechten Verarbeitungszeiten herstellen. Der Fachmann erkennt, dass die Abbindezeit der einzelnen Beispiele durch Erhöhung bzw. Reduzierung der Menge an eingesetztem Katalysatorsalz einfach auf einen gewünschten Wert eingestellt werden kann.

### Vergleichsbeispiele 8 bis 19 (nicht erfindungsgemäß)

Verschiedene Katalysatorsalze gebildet aus den in der Tabelle 2 angegebenen Mengen starker Basen und verschiedenen Carbonsäureanionen wurden in 5,0 Teilen entmineralisiertem Wasser (Ampuwa, pH 5,8) gelöst und gleichermaßen wie in den Beispielen 13 bis 40 zu einer Katalysatorkomponente B verarbeitet.

Jeweils 1 Teil der zuvor beschriebenen Katalysatorkomponenten B und 5 Teile der Basiskomponente A gemäß Beispiel 1 wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Materialien auf Basis von Alkoxysilylpolyethem sind in der Tabelle 2 zusammengefasst.

Wie aus der Tabelle 2 ersichtlich weisen die in den Vergleichsbeispielen 8 bis 19 hergestellten Materialien, in denen als Katalysator jeweils ein aus nicht erfindungsgemäßen Säureanion gebildetes Salz eingesetzt wurde, im Unterschied zu den erfindungsgemäßen Formulierungen inakzeptable Abbindezeiten von mehr als 30 Minuten auf bzw. reagieren nicht.

### Vergleichsbeispiel 20 (nicht erfindungsgemäß)

*(Säurekatalysiertes kondensationsvernetzendes Dentalmaterial auf Basis von Alkoxysilylpolyethern gemäß den Beispielen 3 und 5 der* EP 1 226 808 A2*)*

Ein säurekatalysiertes Dentalmaterial nach dem Stand der Technik auf Basis von Alkoxysilypolyethem bestehend aus einer sauren Katalysatorkomponente B und einer Basiskomponente A wird gemäß Beispielen 3 und 5 der EP 1 226 808 A2 hergestellt und gemischt.

Die Verarbeitungszeit, Abbindezeit und die Abbindezeit nach einem Thermostresstest von einer Woche bei 60°C der gemäß Vergleichsbeispiel 20 hergestellten Zusammensetzung im Vergleich zu dem in Beispiel 9 erhaltenen erfindungsgemäßen Dentalmaterial auf Basis von Alkoxysilylpolyethem ist in der Tabelle 3 wiedergegeben.

Im Vergleich zu den erfindungsgemäßen Dentalmaterialien auf Basis von Alkoxysilylpolyethern zeigt das säurekatalysierte System aus EP 1 226 808 A2 (Beispiele 3 und 5) nach Lagerung im Thermostresstest (eine Woche 60 °C) keine Aushärtung mehr. Dies zeigt, dass die Katalysatorkomponente aus Beispiel 3 der EP 122 6088 A2 instabil ist. Dies führt zu einer Verlängerung der Abbindezeit. Eine unabhängig von der Lagerzeit gleich bleibende Abbindezeit ist allerdings eine der wichtigsten Anforderungen an ein dentales Abformmaterial.

### Beispiele 9a bis 9e (erfindungsgemäß)

Der in Beispiel 9 hergestellten Katalysatorkomponente B wurden verschiedene adstringierende bzw. antibakterielle Zusätze in den in der Tabelle 4 wiedergegebenen Mengen zugegeben und wie in Beispiel 9 beschrieben in Schlauchbeutel (Verbundfolie PE/AI/PE) abgefüllt und aufbewahrt. Jeweils 1 Teil dieser Katalysatorkomponenten wurde mit 5 Teilen der gemäß Beispiel 9 hergestellten Basiskomponente A mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten und Abbindezeit nach einem Thermostresstest von einer Woche bei 60°C der so hergestellten Dentalmaterialien sind in der Tabelle 4 sowie weitere anwendungstechnische Eigenschaften für das in Beispiel 9c erhaltene Dentalmaterial in Tabelle 5 zusammengefasst.

Wie aus der Tabelle 4 hervorgeht hat der Zusatz an adstringierenden bzw. antibakteriellen Zusätze keinen bzw. lediglich einen vernachlässigbar geringen Einfluss auf die Abbindezeit und die Lagerstabilität der erfindungsgemäßen Zusammensetzungen. Wie zudem aus der Tabelle 5 ersichtlich erfüllen die erfindungsgemäßen Dentalmaterialien trotz Zusatz von adstringierenden bzw. antibakteriellen alle Anforderungen an Dentalmaterialien.

### Beispiel 41

### (Herstellung einer Katalysatorkomponente B mit einem Katalysatorsalz gebildet aus einem Kronenether-Alkalimetall-Komplex-Kation und dem 2-Ethylhexansäure-Anion gemäß der ersten Ausführungsform der vorliegenden Erfindung)

1,37 Teile 2-Ethylhexansäure wurden in 5 Teilen entmineralisiertem Wasser (Ampuwa, pH 5,8) gelöst, mit 0,53 Teilen Kaliumhydroxid neutralisiert, mit 2,51 Teilen einen Kronenethers von Typ 18-Crown-6 versetzt und intensiv gemischt, bis sich der Kronenether vollständig gelöst hat. Die Salzlösung wurde in einem Vakuummischer mit 36 Teilen Polypropylenglycol mit einer Molmasse von 4000 g/mol, 51 Teilen Aluminiumhydroxid mit einer mittleren Korngröße von 13 µm und einer BET-Oberfläche von kleiner 1 m²/g und 5 Teilen Kieselsäure mit einer BET-Oberfläche von 130 m²/g für 5 Minuten gemischt. Danach wurde weitere 30 Minuten unter Vakuum homogen gemischt.

Es wurde ein mittelfließendes Material (ISO 4823) erhalten, das die Katalysatorkomponente B des erfindungsgemäßen Dentalmaterials auf Basis von Alkoxysilylpolyethern darstellt. Das Material wurde in einen Schlauchbeutel (Verbundfolie PE/Alu/PE) abgefüllt und aufbewahrt.

### Mischung der Katalysatorkomponente B aus Beispiel 41 und der Basiskomponente A aus Beispiel 1

Ein Teil der zuvor beschriebenen Katalysatorkomponente B und 5 Teile der gemäß Beispiel 1 hergestellten Basiskomponente A wurden mit Hilfe eines elektrischen Austraggerätes aus Schlauchbeuteln (Plug & Press System, Kettenbach GmbH & Co. KG) über einen dynamischen Mischer (Kettenbach GmbH & Co. KG) homogen gemischt.

Die in der Tabelle 6 zusammengefasste Abbindezeit des so hergestellten Dentalmaterials auf Basis von Alkoxysilylpolyethern mit einem Katalysatorsalz aus Kronenether-Kalium-Komplex-Kation mit 2-Ethylhexansäure-Anion weist überraschenderweise eine für kondensationsvemetzende Alkoxysilylpolyether-Systeme hervorragende Aushärtekinetik auf. Es lassen sich schnellabbindende Abformmaterialien mit praxisgerechten Verarbeitungszeiten herstellen.

Der pH-Wert des o. g. erfindungsgemäßen Katalysatorsalzes liegt mit ca. pH 7 im neutralen Bereich, so dass hier eine hervorragende Schleimhautverträglichkeit im Patientenmund gegeben ist.

**Tabelle 1**

| **Zusammensetzung und Abbindezeit der Massen gemäß den Beispielen 1 bis 12 und den Vergleichsbeispielen 1 bis 7** | | | | | | | |
|---|---|---|---|---|---|---|---|
| **erfindungsgemäße Beispiele/ Vergleichsbeispiele** | **starke Base** | **schwache Säure** | **Base (%)** **[mmol/g]** | **Säure (%)** **[mmol/g]** | **pK_{BH+}-Wert Base** **(H₂O)** | **pK_{BH+}-Wert Base** **(MeCN)** | **Abbindezeit** |
| Beispiel 1 | tert-Butylimino-tri(pyrrolidino)phosphoran | 2-Ethylhexansäure | 2,97 [3,20] | 1,37 [6,93] | | 28,4 ²⁾ | < 1min |
| Beispiel 2 | 1-tert-Butyl-2,2,4,4,4-pentakis-(dimethylamino)-2Λ⁵,4Λ⁵-catenadi(phosphazen) | 2-Ethylhexansäure | 3,49 [2,72] | 1,37 [6,93] | | 33,5 ²⁾ | < 1min |
| Beispiel 3 | 1-Ethyl-2,2,4,4,4-pentakis(dimethylamino)-2Λ⁵,4Λ⁵-catenadi(phosphazen) | 2-Ethylhexansäure | 3,22 [2,95] | 1,37 [6,93] | | ²⁾ | < 1 min |
| Beispiel 4 | 1-tert-Butyl-4,4,4-tris(dimethylamino)-2,2-bis-[tris(dimethylamino)phosphoranylidenamino]-2A5,4A5-catenadi(phosphazen) | 2-Ethylhexansäure | 6,02 [1,58] | 1,37 [6,93] | | 41,9 ²⁾ | < 1 min |
| Beispiel 5 | tert-Octylimino-tris(dimethylamino)phosphoran | 2-Ethylhexansäure | 2,76 [3,44] | 1,37 [6,93] | | 26,5 ²⁾ | 5'00" |
| Beispiel 6 | 2,8,9-Triisopropyl-2,5,8,9-tetraaza-1-phosphabicyclo[3.3.3]undecane | 2-Ethylhexansäure | 2,42 [3,92] | 1,37 [6,93] | | 33,63 ³⁾ | 5'30" |
| Beispiel 7 | 1,5-Diazabicyclo[4.3.0]non-5-en | 2-Ethylhexansäure | 1,18 [8,05] | 1,37 [6,93] | | 23,89 ²⁾ | 6'00" |
| Beispiel 8 | 1,1,3,3-Tetramethylguanidin | 2-Ethylhexansäure | 1,09 [8,68] | 1,37 [6,93] | | 23,3 ²⁾ | 6'30" |
| Beispiel 9 | 1,8-Diazabicyclo[5.4.0]undec-7en | 2-Ethylhexansäure | 1,45 [6,57] | 1,37 [6,93] | 12 ⁴⁾ | 24,33 ²⁾ | 7'00" |
| Beispiel 10 | 7-Methyl-1,5,7-triazabicyclo[4.4.0]dec-5-en | 2-Ethylhexansäure | 1,46 [6,53] | 1,37 [6,93] | | 25,44 ²⁾ | 7'30" |
| Beispiel 11 | 2-tert-Butyl-1,1,3,3,-tetramethylguanidin | 2-Ethylhexansäure | 1,63 [5,84] | 1,37 [6,93] | 14 ⁷⁾ | > 20 | 9'30" |
| Beispiel 12 | 1,5,7-Triazabicyclo[4.4.0]dex-5-en | 2-Ethylexansäure | 1,32 [7,18] | 1,37 [6,93] | | 25,98 ²⁾ | 10'00" |
| Vergleichsbeispiel 1 | 2-Methylimidazol | 2-Ethylhexansäure | 0,78 [12,18] | 1,37 [6,93] | 7,80 ¹⁰⁾ | < 20 | > 60 min |
| Vergleichsbeispiel 2 | 4-Methylimidazol | 2-Ethylhexansäure | 0,78 [12,18] | 1,37 [6,93] | 7,45 ¹⁰⁾ | < 20 | > 60 min |
| Vergleichsbeispiel 3 | N-Methylimidazol | 2-Ethylhexansäure | 0,78 [12,18] | 1,37 [6,93] | 6,95 ⁵⁾ | < 20 | > 60 min |
| Vergleichsbeispiel 4 | Triethanolamin | 2-Ethylhexansäure | 1,42 [6,70] | 1,37 [6,93] | 7,77 ⁹⁾ | < 20 | keine Reaktion |
| Vergleichsbeispiel 5 | 1,2,2,6,6-Pentamethylpiperidin | 2-Ethylhexansäure | 1,48 [6,44] | 1,37 [6,93] | 11,25 ⁶⁾ | 18,62 ²⁾ | keine Reaktion |
| Vergleichsbeispiel 6 | Tributylamin | 2-Ethylhexansäure | 1,76 [5,39] | 1,37 [6,93] | | 18,09 ²⁾ | keine Reaktion, Phasentrennung unlöslich |
| Vergleichsbeispiel 7 | Triphenylphosphin | 2-Ethylhexansäure | 2,49 [3,81] | 1,37 [6,93] | 2,73 ⁸⁾ | < 20 | keine Reaktion, Phasentrennung unlöslich |

| | | | | | | | |
|---|---|---|---|---|---|---|---|
| ²⁾ Quelle: ChemFiles: Strong and Hindered Bases in Organic Synthesis, Vol.3, No. 1, Fluka ³⁾ Topics in Current Chemistry, Vol. 223, John G. Verhade "P(RNCH2CH2)3N: Very Strong Non-lonic Bases Useful in Organic Synthesis" ⁴⁾ http://www.cem.msu.edu/-reusch/VirtualText/suppmnt2.htm ⁵⁾ Quelle: http://www.zirchrom.com/organic.htm, "Dissociation constants of organic acids and bases" ⁶⁾ Quelle: H.Z. Sommer, H.I. Lipp, L.L. Jackson, J. Org. Chem., Vol. 36, No.6 (1971) 824 ⁷⁾ 50 %ig in Ethanol, Quelle: DHR Barton, J.D. Elliot, SD Gero, J.Chem.Soc.Perkin Trans.I 1982, 2085 ⁸⁾ 50 %ig in Ethanol, Quelle: W.A. henderson, C.A.Strueli, J.Am.Chem.Soc 1960, 82,5791 ⁹⁾ Quelle: H.K. Hall, J. Am. Chem. Soc. 1957, 79, 5441 ¹⁰⁾ Quelle: M Schmidt am Busch, E.W. Knapp ChemPhysChem 2004, 5, 1513-1522 | | | | | | | |

**Tabelle 2**

| **Zusammensetzung und Abbindezeit der Massen gemäß den Beispielen 13 bis 40 und den Vergleichsbeispielen 8 bis 19** | | | | | | | | |
|---|---|---|---|---|---|---|---|---|
| **erfindungsgemäße Beispiele/ Vergleichsbeispiele ¹⁾** | **starke Base** | **schwache Säure** | **Base (%)** **[mmol/g]** | **Säure (%)** **[mmol/g]** | **pK_{BH+}-Wert** **(MeCN)** | **Anzahl der C-Atome der Base längsten C-Kette*** | **Anzahl der Substituenten in der längsten C-Kette der Säure²⁾** | **Abbindezeit** |
| Beispiel 13 | 1,1,3,3-Tetramethyl-guanidin | 2,2-Dimethylnonansäure | 1.09 [8,68] | 1,77 [5,37] | 23,3³⁾ | 8 | (α,α')** 2 | 4'00" |
| Beispiel 14 | 1,5-Diazabicyclo[4.3.0] non-5-en | 2,2-Dimethylhexansäure | 1,18 [8.05] | 1,37 [6,93] | 23,89 ³⁾ | 5 | (α,α')** 2 | 4'45" |
| Bespiel 15 | 1,1,3,3-Tetramethylguanidin | 2,2-Dimethylhexansäure | 1,09 [8,68] | 1,37 [6,93] | 233 ³⁾ | 5 | (α,α')** 2 | 5'00" |
| Beispiel 16 | 1,8-Diazabicyclo[5.4.0] undec-7en | 2,2-Dimethylnonansäure | 1,45 [6,57] | 1,77 [5,37] | 24,33 ³⁾ | 8 | (α,α')** 2 | 5'00" |
| Beispiel 17 | 1,5-Diazabicyclo[4.3.0] non-5-en | 2,2-Dimethylnonansäure | 1,09 [8,05] | 1.77 [5,37] | 23,89 ³⁾ | 8 | (α,α')** 2 | 5'00" |
| Beispiel 18 | 1,8-Diazabicyclo[5.4.0] undec-7en | 2,2-Dimethylhexansäure | 1,45 [6,57] | 1,37 [6,93] | 24,33 ³⁾ | 5 | (α,α')** 2 | 5'30" |
| Beispiel 19 | 1,8-Diazabicyclo[5.4.0] undec-7en | 1-Methyl-1-cyclohexancarbonsäure | 1,45 [6,57] | 1,35 [7,03] | 24,33 ³⁾ | 6 | (α,α')** 2 | 6'00" |
| Beispiel 20 | 1,8-Diazabicyclo[5.4.0] undec-7en | 2-Methylhexansäure | 1,45 [6,57] | 1,24 [7,68] | 24,33 ³⁾ | 5 | (α)** 1 | 6'00" |
| Beispiel 21 | 1,8-Diazabicyclo[5.4.0] undec-7en | 2,2-Dimethylbuttersäure | 1,45 [6,57] | 1,10 [8,61] | 24,33 ³⁾ | 3 | (α,α')** 2 | 6'30" |
| Beispiel 22 | 1,8-Diazabicyclo[5.4.0] undec-7en | 2,2-Dimethylvaleriansäure | 1,45 [6,57] | 1,24 [7,68] | 24,33 ³⁾ | 4 | (α,α')** 2 | 6'30" |
| Beispiel 23 | 1,8-Diazabicyclo[5.4.0] undec-7en | 2-Ethylhexansäure | 1,45 [6.57] | 1,37 [6,93] | 24.33 ³⁾ | 5 | (α)** 1 | 7'00" |
| Beispiel 24 | 1,8-Diazabicyclo[5.4.0] undec-7en | 2-Butyloctansäure | 1,45 [6,57] | 1,90 [4,99] | 24,33 ³⁾ | 7 | (α)** 1 | 7'00" |
| Beispiel 25 | 1,8-Diazabicyclo[5.4.0] undec-7en | 2-Ethylbuttersäure | 1,45 [6,57] | 1,10 [8,61] | 24.33 ³⁾ | 3 | (α)** 1 | 7'00" |
| Beispiel 26 | 1,8-Diazabicyclo[5.4.0] undec-7en | 2-Hexyldecansäure | 1,45 [6,57] | 2,44 [3,90] | 24,33 ³⁾ | 9 | (α)** 1 | 7'00" |
| Beispiel 27 | 1,8-Diazabicyclo[5.4.0] undec-7en | 2-Methylbuttersäure | 1,45 [6,57] | 0,97 [9,79] | 24,33 ³⁾ | 3 | (α)** 1 | 7'30" |
| Beispiel 28 | 1,8-Diazabicyclo[5.4.0] undec-7en | Pivalinsäure | 1,45 [6,57] | 0,97 [9,79] | 24.33 ³⁾ | 2 | (α,α')** 2 | 7'30" |
| Beispiel 29 | 1,8-Diazabicyclo[5.4.0] undec-7en | Decansäure | 1,45 [6,57] | 1,64 [5,80] | 24,33 ³⁾ | 9 | 0 | 8'00" |
| Beispiel 30 | 1,8-Diazabicyclo[5.4.0] undec-7en | 4-Methylhexansäure | 1,45 [6,57] | 1,24 [7,68] | 24,33 ³⁾ | 5 | (γ)** 1 | 8'00" |
| Beispiel 31 | 1,8-Diazabicyclo[5.4.0] undec-7en | Octansäure | 1,45 [6,57] | 1,37 [6,93] | 24,33 ³⁾ | 7 | 0 | 8'00" |
| Beispiel 32 | 1,8-Diazabicyclo[5.4.0] undec-7en | Hexansäure | 1,45 [6,57] | 1,10 [8,61] | 24,33 ³⁾ | 5 | 0 | 8'00" |
| Beispiel 33 | 1,8-Diazabicyclo[5.4.0] undec-7en | Bicyclo[2.2.1]-5-heptan-2-carbonsäure | 1,45 [6,57] | 1,33 [6,57] | 24,33 ³⁾ | 6 | 2 | 8'00" |
| Beispiel 34 | 1,8-Diazabicyclo[5.4.0] undec-7en | Myristinsäure | 1,45 [6,57] | 2,17 [4,38] | 24,33 ³⁾ | 13 | 0 | 8'00" |
| Beispiel 35 | 1,8-Diazabicyclo[5.4.0] undec-7en | 2-Propylpentansäure | 1,45 [6,57] | 1,37 [6,93] | 24.33 ³⁾ | 4 | (α)** 1 | 8'30" |
| Beispiel 36 | 1,8-Diazabicyclo[5.4.0] undec-7en | Önanthsäure | 1,45 [6,57] | 1,24 [7,69] | 24,33 ³⁾ | 6 | 0 | 9'00" |
| Beispiel 37 | 1,8-Diazabicyclo[5.4.0] undec-7en | Cyclohexancarbonsäure | 1,45 [6,57] | 1,22 [7,80] | 24,33 ³⁾ | 6 | (α,α')** 2 | 9'00" |
| Beispiel 38 | 1,8-Diazabicyclo[5.4.0] undec-7en | Isobuttersäure | 1,45 [6,57] | 0,84 [11,35] | 24,33 ³⁾ | 3 | (β)** 1 | 9'30" |
| Beispiel 39 | 1,8-Diazabicyclo[5.4.0] undec-7en | Ölsäure | 1,45 [6,57] | 2,68 [3,54] | 24,33 ³⁾ | 15 | 0 | 10'30" |
| Beispiel 40 | 1,8-Diazabicyclo[5.4.0] undec-7en | Stearinsäure (Octadecansäure) | 1,45 [6,57] | 2,70 [3,52] | 24,33 ³⁾ | 17 | 0 | 10'30" |
| Vergleichsbeispiel 8 | 1,8-Diazabicyclo[5.4.0] undec-7en | Salzsäure | 1,45 [6,57] | 0,35 [27,43] | 24,33 ³⁾ | -* | -* | > 30 min |
| Vergleichsbeispiel 9 | 1,8-Diazabicyclo[5.4.0] undec-7en | Benzoesäure | 1,45 [6,57] | 1,16 [8,19] | 24,33 ³⁾ | 6 | 1 | >30 min |
| Vergleichsbeispiel 10 | 1,8-Diazabicyclo[5.4.0] undec-7en | Essigsäure | 1,45 [6,57] | 0,57 [16,65] | 24.33 ³⁾ | 1 | 0 | >30 min |
| Vergleichsbeispiel 11 | 1,8-Diazabicyclo[5.4.0] undec-7en | Acrylsäure | 1,45 [6,57] | 0,68 [13,88] | 24,33 ³⁾ | 2 | 0 | >30 min |
| Vergleichsbeispiel 12 | 1,8-Diazabicyclo[5.4.0] undec-7en | Ameisensäure | 1,45 [6,57] | 0,44 [21,72] | 24,33 ³⁾ | 0 | - | >60 min |
| Vergleichsbeispiel 13 | 1,8-Diazabicyclo[5.4.0] undec-7en | Salicylsäure | 1,45 [6,57] | 1,31 [7,24] | 24,33 ³⁾ | 6 | 1 | keine Reaktion |
| Vergleichsbeispiel 14 | 1,8-Diazabicyclo[5.4.0] undec-7en | Acetylsalicylsäure | 1,45 [6,57] | 1,71 [5,55] | 24,33 ³⁾ | 6 | 1 | keine Reaktion |
| Vergleichsbeispiel 15 | 1,8-Diazabicyclo[5.4.0] undec-7en | Ascorbinsäure | 1,45 [6,57] | 1,67 [5,68] | 24,33 ³⁾ | - | - | keine Reaktion |
| Vergleichsbeispiel 16 | 1,8-Diazabicyclo[5.4.0] undec-7en | Schwefelsäure | 1,45 [6,57] | 0,93 [10,20] | 24,33 ³⁾ | - | - | keine Reaktion |
| Vergleichsbeispiel 17 | 1,8-Diazabicyclo[5.4.0] undec-7en | Phosphorsäure | 1,45 [6,57] | 0,93 [10,21] | 24,33 ³⁾ | - | - | keine Reaktion |
| Vergleichsbeispiel 18 | 1,8-Diazabicyclo[5.4.0] undec-7en | o-Phthalsäure | 1,45 [6,57] | 1,58 [6,02] | 24,33 ³⁾ | 6 | 1 | keine Reaktion |
| Vergleichsbeispiel 19 | 1,8-Diazabicyclo[5.4.0] undec-7en | p-Toluolsulfonsäure | 1,45 [6,57] | 1,81 [5,26] | 24,33 ³⁾ | - | - | keine Reaktion |

| | | | | | | | | |
|---|---|---|---|---|---|---|---|---|
| ²⁾ 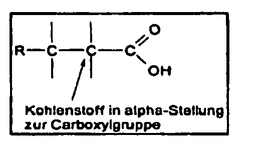 ³⁾ Quelle: Sigma-Aldrich, ChemFiles Vol.3, No. 1 * ohne Carboxylgruppe ** in Stellung zur Carboxylgruppe | | | | | | | | |

**Tabelle 3**

| **Verarbeitungs-, Abbindezeiten und Stabilitäten von Dentalmaterialien auf Basis von Alkoxysilylpolyethern im Vergleich zu den Beispielen 3 und 5 der** EP 1226808 A2 | | | |
|---|---|---|---|
| **Abformmaterial** | **Verarbeitungszeit ¹⁾** | **Abbindezeit ²⁾** | **Stabilität: Abbindezeit nach einer Woche 60°C ³⁾** |
| Beispiel 9 | 2,00 min | 7,00 min | 7,00 min |
| Vergleichsbeispiel 20 Beispiel 3 und 5 EP 122 6808 A2 | 0,50 min | 5,00 | > 15,00 min |

| | | | |
|---|---|---|---|
| ¹⁾ gemäß ISO 4823 (Ausgabe 1992) ²⁾ bestimmt über Rückstellung nach der Verformung nach ISO 4823 (Ausgabe 1992) ³⁾ Einlagerung in verschlossene Schlauchbeuteln aus PE/AI/PE-Verbundfolie, Messung siehe unter ²⁾ | | | |

**Tabelle 4**

| **Erfindungsgemäße Beispiele für adstringierende bzw. antibakterielle Zusätze gemäß Beispiel 9** | | | | |
|---|---|---|---|---|
| **Beispiel** | **adstringierender bzw. antibakterieller Zusatz** | **Menge Zusatz** **(Teile)** | **Abbindezeit** **[min]** | **Stabilität Abbindezeit nach 1 Woche 60°C** **[min]** |
| 9a | kein | 0,0 | 7,00 | 7,00 |
| 9b | Triclosan | 0,6 | 7,00 | 7,00 |
| 9c | Epinephrin | 0,6 | 7,00 | 7,00 |
| 9d | Chlorhexidine | 0,6 | 7,50 | 7,50 |
| 9e | Hexetidine | 0,6 | 7,25 | 7,75 |

**Tabelle 5**

| **Anwendungstechnische Eigenschaften der Zusammensetzungen gemäß den Beispielen 9 und 9c** | | |
|---|---|---|
| | **Beispiel 9** | **Beispiel 9c** **Zusatz von Epinephrin** |
| **Konsistenz ¹⁾ Katalysatorkomponente B** | 39 mm | 38 mm |
| **Konsistenz ¹⁾ Basiskomponente A** | 35 mm | 35 mm |
| **Konsistenz der Mischung ²⁾** | 36 mm | 36 mm |
| **Lineare Maßänderung ³⁾** | - 0,45 % | - 0,45 % |
| **Shore A-Härte sofort nach Abbindeende ⁴⁾** | 56 | 54 |
| **Shore A-Härte sofort nach 15 Stunden Lagerung ⁴⁾** | 63 | 63 |
| **Kontaktwinkel ⁵⁾** | 40 | 40 |
| **Rückstellung nach der Verformung ⁶⁾** | 98,9 % | 98,6 % |

| | | |
|---|---|---|
| ¹⁾ in Anlehnung an ISO 4823, Konsistenz der Mischung, Belastungsgewicht 500 g, Belastungsdauer 15 s ²⁾ gemäß ISO 4823, Konsistenz der Mischung, Belastungsgewicht 1500 g, Belastungsdauer 5 s ³⁾ gemäß ISO 4823 ⁴⁾ gemäß DIN 53505 mit digitalem Shore-Härteprüfgerät Fa. Zwick ⁵⁾ gemessen nach der Methode des liegenden Tropfens, Kontaktwinkelmeßsystem Fa. Krüss G40, initialer Kontaktwinkel (Prüfkörperalter: Aufbringen des Tropfens 45 Sek. nach Mischbeginn), Messzeitpunkt: 30 Sek. nach Aufbringen des Tropfens, Verwendung von entmineralisiertem Wasser. ⁶⁾ gemäß ISO 4823 | | |

**Tabelle 6**

| **Abbindezeit der Zusammensetzung gemäß Beispiel 41** | | | | | |
|---|---|---|---|---|---|
| **Beispiel** | **Kronenether-Alkalimetall-Komplex-Kation** | **Anion der schwache Säure** | **(%) Kronenether-Komplex** **[mmol/g]** | **(%) Anion der schwachen Säure** **[mmol/g]** | **Abbindezeit** |
| Beispiel 41 | 18-Crown-6-KaliumKomplex-Kation | 2-Ethylhexansäure-Anion | 2,88 [3,30] | 1,37 [6,93] | 7'00" |

## Patentansprüche

1. Kondensationsvernetzendes Dentalmaterial enthaltend:
a) wenigstens einen alkoxysilylfunktionellen Polyether und
b) wenigstens einen Katalysator,
**dadurch gekennzeichnet, dass** der wenigstens eine Katalysator b) ein Salz ist, welches gebildet ist aus wenigstens einem aus der aus
- Komplexen von Alkalimetall- oder Ammonium-Kationen mit Kronenethern und/oder Kryptanden,
- Tetraalkyl-, Tetraaryl-, Trialkylaryl-, Dialkyldiaryl-, Monoalkyltriarylammonium-Kationen, Tetraalkyl-, Tetraaryl-, Trialkylaryl-, Dialkyldiaryl-, Monoalkyltriarylphosphonium-Kationen, Tetraalkyl-, Tetraaryl-, Trialkylaryl-, Dialkyldiaryl-, Monoalkyltriarylarsonium-Kationen, Tetraalkyl-, Tetraaryl-, Trialkylaryl-, Dialkyldiaryl-, Monoalkyltriarylstibonium-Kationen,
- durch Protonierung einer Base gebildete Kationen, wobei die Base einen in Acetonitril gemessenen pK_{BH+}-Wert von mindestens 20 aufweist,
und Kombinationen hiervon bestehenden Gruppe ausgewählten Kation und wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist.

2. Kondensationsvernetzendes Zweikomponenten-Dentalmaterial mit einer Komponente A enthaltend
a) wenigstens einen alkoxysilylfunktionellen Polyether
und einer Komponente B enthaltend
b) wenigstens einen Katalysator und
c) Wasser,
wobei der wenigstens eine Katalysator b) ein Salz ist, welches gebildet ist aus wenigstens einem aus der aus
- Komplexen von Alkalimetall- oder Ammonium-Kationen mit Kronenethern und/oder Kryptanden,
- Tetraalkyl-, Tetraaryl-, Trialkylaryl-, Dialkyldiaryl-, Monoalkyltriarylammonium-Kationen, Tetraalkyl-, Tetraaryl-, Trialkylaryl-, Dialkyldiaryl-, Monoalkyltriarylphosphonium-Kationen, Tetraalkyl-, Tetraaryl-, Trialkylaryl-, Dialkyldiaryl-, Monoalkyltriarylarsonium-Kationen, Tetraalkyl-, Tetraaryl-, Trialkylaryl-, Dialkyldiaryl-, Monoalkyltriarylstibonium-Kationen,
- durch Protonierung einer Base gebildete Kationen, wobei die Base einen in Acetonitril gemessenen pK_{BH+}-Wert von mindestens 20 aufweist,
und Kombinationen hiervon bestehenden Gruppe ausgewählten Kation und wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist.

3. Kondensationsvernetzendes Dentalmaterial nach Anspruch 1, **dadurch gekennzeichnet, dass** dieses wenigstens einen verstärkenden Füllstoff d₁) mit einer BET-Oberfläche von mindestens 50 m²/g und/oder wenigstens einen nicht verstärkenden Füllstoff d₂) mit einer BET-Oberfläche von weniger als 50 m²/g enthält.

4. Kondensationsvernetzendes Zweikomponenten-Dentalmaterial nach Anspruch 2, **dadurch gekennzeichnet, dass** dieses in der Komponente A und/oder in der Komponente B wenigstens einen verstärkenden Füllstoff d₁) mit einer BET-Oberfläche von mindestens 50 m²/g und/oder wenigstens einen nicht verstärkenden Füllstoff d₂) mit einer BET-Oberfläche von weniger als 50 m²/g enthält.

5. Dentalmaterial nach einem der vorhergehenden Ansprüche, **dadurch gekennzeichnet, dass** das Kation des Katalysatorsalzes b) ein Komplex aus einem oder mehreren Lithium-, Natrium-, Kalium-, Rubidium-, Cäsium- und/oder Ammonium-Ionen und einem oder mehreren aus der aus ein 15-Crown-5, 18-Crown-6, Dibenzo-18-crown-6, Dibenzo-21-crown-7, Dibenzo-24-crown-8, Dibenzo-30-crown-10, 1,4,10-Trioxa-7,13-diazacyclopentadecan, 4,7,13,18-Tetraoxa-1,10-diazabicyclo[8.5.5]eicosan, 1,4,10,13-Tetraoxa-7,16-diazacyclooctadecan, 3,6,9,14-Tetrathiabicyclo[9.2.1]tetradeca-11,13-dien, 1,4,7,10-Tetrathiacyclododecan, 1,5,9,13-Tetrathiacyclohexadecan-3,11-diol, 1,5,9-Triazacyclododecan, 1,4,7-Triazacyclononan, 1,4,7,10,13,16-Hexamethyl-1,4,7,10,13,16-hexaazacyclooctadecan, 1,4,10-Trioxa-7,13-diazacyclopentadecan, 4,7,13,18-Tetraoxa-1,10-diazabicyclo[8.5.5]eicosan, 1,4,10,13-Tetraoxa-7,16-diazacyclooctadecan, Dibenzo-21-crown-7, Dibenzo-24-crown-8, Dibenzo-30-crown-10, 18-Crown-6, 15-Crown-5, 3,6,9,14-Tetrathiabicyclo[9.2.1]tetradeca-11,13-dien, 1,4,7,10-Tetrathiacyclododecan, 1,5,9,13-Tetrathiacyclohexadecan-3,11-diol, 1,5,9-Triazacyclododecan und 1,4,7-Triazacyclononan bestehenden Gruppe ausgewählten Kronenether und/oder Kryptanden ist.

6. Dentalmaterial nach einem der Ansprüche 1 bis 4, **dadurch gekennzeichnet, dass** das Kation des Katalysatorsalzes b) ein aus der aus Tetramethylammonium-, Tetraethylammonium-, Tetrapropylammonium-, Tetrabutylammonium-, Tetrapentylammonium-, Tetrahexylammonium-, Tetraheptylammonium-, Tetraoctylammonium-, Tetranonylammonium-, Tetradecylammonium-, Tetramethylphosphonium-, Tetraethylphosphonium-, Tetrapropylphosphonium-, Tetrabutylphosphonium-, Tetrapentylphosphonium-, Tetrahexylphosphonium-, Tetraheptylphosphonium-, Tetraoctylphosphonium-, Tetranonylphosphonium-, Tetradecylphosphonium-, Tetramethylarsonium-, Tetraethylarsonium-, Tetrapropylarsonium-, Tetrabutylarsonlum-, Tetrapentylarsonium-, Tetrahexylarsonium-, Tetraheptylarsonium-, Tetraoctylarsonium-, Tetranonylarsonium-, Tetradecylarsonium-, Tetramethylstibonium-, Tetraethylstibonium-, Tetrapropylstibonium-, Tetrabutylstibonium-, Tetrapentylstibonium-, Tetrahexylstibonium-, Tetraheptylstibonium-, Tetraoctylstibonium-, Tetranonylstibonium-, Tetradecylstibonium-, Lauryltrimethylammonium-, Myristyltrimethylammonium-, Cetyltrimethylammonium-, Stearyltrimethylammonium-, Lauralkonium-, Myristalkonium-, Cetalkonium-, Stearalkonium-, Cetylethyldimethylammonium-, Benzyltriethylammonium-, Benzalkonium-Kationen und Kombinationen hiervon bestehenden Gruppe ausgewähltes Ion ist.

7. Dentalmaterial nach einem der Ansprüche 1 bis 4, **dadurch gekennzeichnet, dass** das Kation des Katalysatorsalzes b) ein durch Protonierung einer Base mit einem in Acetonitril gemessenen pK_{BH+}-Wert von mindestens 21 gebildetes Ion ist.

8. Dentalmaterial nach Anspruch 7, **dadurch gekennzeichnet, dass** das Katalysatorsalz b) aus einer Base gebildet ist, welche wenigstens eine Struktureinheit gemäß der allgemeinen Formel I 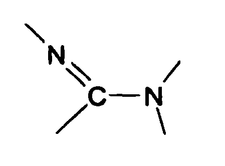 und/oder gemäß der allgemeinen Formel II 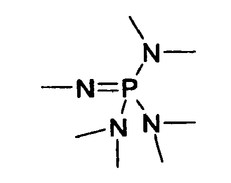 und/oder gemäß der allgemeinen Formel III 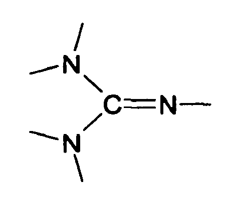 aufweist.

9. Dentalmaterial nach Anspruch 7 oder 8, **dadurch gekennzeichnet, dass** als Kation des Katalysatorsalzes b) eine protonierte, aus der aus 1,1,3,3-Tetramethylguanidin, Diazabicyclo[5.4.0]undec-7-en, 1,5-Diazabicyclo[4.3.0]non-5-en, tert-Butylimino-tris(dimethylamino)phosphoran, tert-Butyliminotri(pyrrolidino)-phosphoran, tert-Octylimino-tris(dimethylamino)phosphoran, 2-tert-Butylimino-2-diethylamino-1,3-dimethyl-perhydro- 1,3,2-diazaphosphorin, 2-tert-Butylimino-2-diethylamino-1,3-dimethyl-perhydro- 1,3,2-diazaphosphorin auf Polystyrol, 1-tert-Butyl-2,2,4,4,4-pentakis(diethylamino)-2A5, 4A5-catenadi(phosphazen), 1-Ethyl-2,2,4,4,4-pentakis(diethylamino)-2Λ5, 4Λ5-catenadi(phosphazen), 1-tert-Butyl-4,4,4-tris(dimethylamino)-2,2-bis[tris(dimethylaminor phosphoranylidenamino]-2Λ⁵,4Λ⁵-catenadi(phosphazen), 1-tert-Octyl-4,4,4-tris(dimethylamino)-2,2-bis[tris(dimethylamino)-phosphoranylidenamino]-2Λ⁵,4Λ⁵-catenadi(phosphazen), 2,8,9-Triisobutyl-2,5,8,9-tetraaza-1-phosphabicyclo[3.3.3]undecan, 2,8,9-Triisopropyl-2,5,8,9-tetraaza-1-phosphabicyclo[3.3.3]undecan, 2,8,9-Trimethyl-2,5,8,9-tetraaza-1-phosphabicyclo[3.3.3]undecan, 1,8-Bis(tetramethylguanidino)naphthalen, 2-tert-Butyl-1,1,3,3-tetramethylguanidin, 1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählte Base, bevorzugt eine protonierte, aus der aus tert-Butyliminotri(pyrrolidino)phosphoran, 1-tert-Butyl-2,2,4,4,4-pentakis(diethylamino)-2Λ5, 4Λ5-catenadi(phosphazen), 1-tert-Butyl-4,4,4-tris(dimethylamino)-2,2-bis[tris(dimethylamino)-phosphoranylidenamino]-2Λ⁵,4Λ⁵-catenadi(phosphazen), tert-Octylimino-tris(dimethylamino)phosphoran, 2,8,9-Tri-isopropyl-2,5,8,9-tetraaza-1-phosphabicyclo[3.3.3]undecan, 1,5-Diazabicyclo[4.3.0]non-5-en, 1,1,3,3-Tetramethylguanidin, Diazabicyclo[5.4.0]undec-7-en, 7-Methyl-1,5,7-triazabicyclo(4.4.0)dec-5-en, 2-tert-Butyl-1,1,3,3-tetra-methylguanidin, 1,5,7-Triazabicyclo(4.4.0)dec-5-en und/oder 1,8-Bis(tetramethylguanidino)naphthalen bestehenden Gruppe ausgewählte Base eingesetzt wird.

10. Dentalmaterial nach einem der vorhergehenden Ansprüche, **dadurch gekennzeichnet, dass** das Anion des Katalysatorsalzes b) ein Anion einer verzweigten Carbonsäure mit einer Länge der an der Carboxylgruppe vorgesehenen (Cyclo)Alkylkette von wenigstens 3 Kohlenstoffatomen oder einer unverzweigten Carbonsäure mit einer Länge der an der Carboxylgruppe vorgesehenen (Cyclo)Alkylkette von wenigstens 5 Kohlenstoffatomen ist.

11. Dentalmaterial nach einem der vorhergehenden Ansprüche, **dadurch gekennzeichnet, dass** das Anion des Katalysatorsalzes b) eine deprotonierte gesättigte und/oder ungesättigte (cyclo)aliphatische Carbonsäure ist, deren (Cyclo)Alkylkette in α-Stellung zu der Carboxylgruppe wenigstens eine Verzweigung aufweist.

12. Dentalmaterial nach einem der vorhergehenden Ansprüche, **dadurch gekennzeichnet, dass** das Anion des Katalysatorsalzes b) ein aus der aus deprotonierten 2,2-Dialkylalkansäuren, 3,3-Dialkylalkansäuren, 4,4-Dialkylalkansäuren, 2,3-Dialkylalkansäuren, 2,4-Dialkylalkansäuren, 3,4-Dialkylalkansäuren, 2,2-Dialkylalkensäuren, 3,3-Dialkylalkensäuren, 4,4-Dialkylalkensäuren, 2,3-Dialkylalkensäuren, 2,4-Dialkylalkensäuren, 3,4-Dialkylalkensäuren, 2,2-Dialkylalkinsäuren, 3,3-Dialkylalkinsäuren, 4,4-Dialkylalkinsäuren, 2,3-Dialkylalkinsäuren, 2,4-Dialkylalkinsäuren, 3,4-Dialkylalkinsäuren, 2-Monoalkylalkansäuren, 3-Monoalkylalkansäuren, 4-Monoalkylalkansäuren, 2,2-Dialkylhexansäuren, bevorzugt 2,2-Dialkylnonan-säure, 2,2-Dimethyl-decansäure, 2,2-Diethyldecansäure, 2,2-Dipropyldecan-säure, 2,2-Dibutyl-decansäure, 2,2-Dimethylnonansäure, 2,2 Diethylnonansäure, 2,2-Dipropylnonansäure, 2,2-Dibutylnonansäure, 2,2-Dimethyloctansäure, 2,2-Diethyloctansäure, 2,2-Dipropyloctansäure, 2,2-Dibutyloctansäure, 2,2-Dimethylheptansäure, 2,2-Diethylheptansäure, 2,2-Dipropylheptansäure, 2,2-Dibutylheptansäure, 2,2-Dimethylhexansäure, 2,2-Diethylhexansäure, 2,2-Dipropylhexansäure, 2,2-Dibutylhexansäure, 2-Butyloctansäure, 2-Hexyl-decansäure, 2-Propylpentansäure, 1-Methyl-1-cyclohexancarbonsäure, 2,2-Dimethylbuttersäure, 2,2-Dimethylvaleriansäure, 3,5,5-Trimethylhexansäure, 2-Ethylhexansäure, Decansäure, Octansäure, Hexansäure und Önanthsäure, bestehenden Gruppe ausgewähltes Ion ist.

13. Dentalmaterial nach einem der vorhergehenden Ansprüche, **dadurch gekennzelichnet, dass** dieses, bezogen auf die Gesamtmischung, 0,001 bis 1 mmol/g wenigstens eines Katalysators b) enthält.

14. Dentalmaterial nach einem der vorhergehenden Ansprüche, **dadurch gekennzeichnet, dass** das eingesetzte Katalysatorsalz in der Polyethermatrix eine hinreichend große Löslichkeit aufweist, um in einer auf die Gesamtmischung bezogenen Menge von 0,001 bis 1 mmol/g eingesetzt eine Aushärtung des Dentalmaterials bestimmt durch Rückstellung nach Verformung gemäß ISO 4823 (Ausgabe 1992) in weniger gleich 30 Minuten für eine dentale Abformmasse zu erzielen.

15. Dentalmaterial nach Anspruch 2, **dadurch gekennzeichnet, dass** dieses als Katalysator b) wenigstens ein Salz von 1,8-Diazabicyclo[5.4.0]undec-7-en, 1,5-Diazabicyclo[4.3.0]non-5-en und/oder 1,1,3,3-Tetramethyl-guanidin mit 2-Ethylhexansäure enthält.

16. Dentalmaterial nach einem der vorhergehenden Ansprüche, **dadurch gekennzeichnet, dass** dieses neben einer oder mehreren Salzen gemäß einem der Ansprüche 1 bis 15 keinen weiteren Katalysator, insbesondere keine metallorganischen Verbindungen, keine Schwermetallcarboxylatsalze, tertiären Amine und freien Säuren, enthält.

17. Dentalmaterial nach einem der vorhergehenden Ansprüche, **dadurch gekennzeichnet, dass** der wenigstens eine alkoxysilylfunktionelle Polyether a) als dritte Struktureinheit jeweils an den terminalen Alkoxysilylgruppen angeordnete Alkylenspacer, welche besonders bevorzugt C₁-C₆-Alkylgruppen sind, und als vierte Struktureinheit 0 bis 8 mmol/g Urethangruppen und/oder 0 bis 8 mmol/g Harnstoffgruppen aufweist.

18. Dentalmaterial nach Anspruch 17, **dadurch gekennzeichnet, dass** der wenigstens eine alkoxysilylfunktionelle Polyether a) innerhalb der Polymerkette keine Urethan- und/oder Harnstoffgruppen enthält und an jedem Kettenende höchstens eine bzw. nicht mehr als eine Urethan und/oder Harnstoffgruppe und höchstens eine bzw. nicht mehr als eine Methylenspacergruppe trägt

19. Dentalmaterial nach Anspruch 17, **dadurch gekennzeichnet, dass** die einzelnen Struktureinheiten des wenigstens einen Polyethers a) gemäß 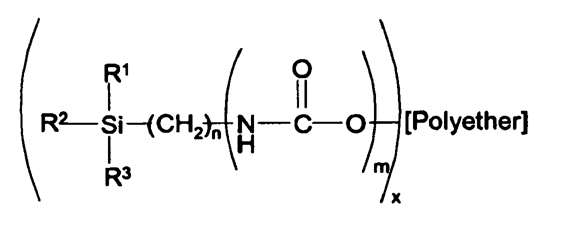 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist/sind
und/oder 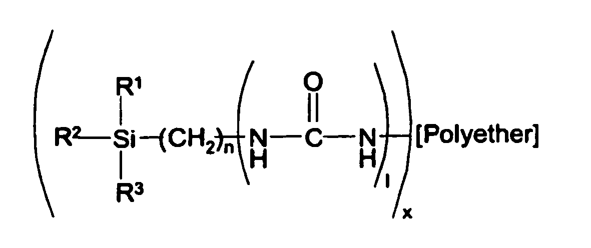 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie l=0 oder 1, besonders bevorzugt l=1 ist/sind,
angeordnet sind.

20. Dentalmaterial nach Anspruch 19, **dadurch gekennzeichnet, dass** n gleich 1 ist.

21. Dentalmaterial nach einem der vorhergehenden Ansprüche, **dadurch gekennzeichnet, dass** dieses wenigstens einen Wasserfänger g), vorzugsweise einen aus der aus Alkoxysilanen, Titanaten, Zirkonaten, Zeolithen, Aluminiumsulfat, wasserfreiem Calciumsulfat, Blaugel, Oxazolidinen, Alkoxysilanen bestehenden Gruppe und besonders bevorzugt einen aus der aus Vinyltrimethoxysilan, N-Trimethoxysilylmethyl-O-methylcarbamat und 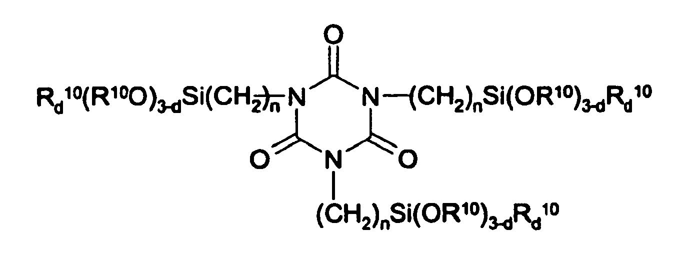 worin n = 1 bis 6, bevorzugt n = 1 oder 3, besonders bevorzugt n = 1,
d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, ist
bestehenden Gruppe ausgewählten Wasserfänger, enthält.

22. Dentalmaterial nach einem der vorhergehenden Ansprüche, **dadurch gekennzeichnet, dass** dieses wenigstens einen Pastenbildner h), vorzugsweise einen aus der aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden, Glycerin und Poly(meth)acrylsäuren bestehenden Gruppe ausgewählten Pastenbildner enthält.

23. Mischung erhältlich durch Vermischen der Komponenten A und B des Zweikomponenten-Dentalmaterials nach einem der Ansprüche 2 bis 22, **dadurch gekennzeichnet, dass** die Basiskomponente A mit der Katalysatorkomponente B in einem Verhältnis von 1:1 bis 20:1 vermischt wird.

24. Verwendung eines Dentalmaterials nach einem der Ansprüche 1 bis 23 in der Dentalmedizin und/oder Dentaltechnik.

## Claims

1. A condensation cross-linking dental material containing:
a) at least one polyether with an alkoxysilyl function; and
b) at least one catalyst;
**characterized in that** the at least one catalyst b) is a salt which is formed from at least one cation selected from the group consisting of:
- complexes of alkali metal or ammonium cations with crown ethers and/or cryptands;
- tetraalkyl, tetraaryl, trialkylaryl, dialkyldiaryl, monoalkyltriaryl-ammonium cations, tetraalkyl, tetraaryl, trialkylaryl, dialkyldiaryl, monoalkyltriaryl-phosphonium cations, tetraalkyl, tetraaryl, trialkylaryl, dialkyldiaryl, monoalkyltriaryl-arsonium cations, tetraalkyl, tetraaryl, trialkylaryl, dialkyldiaryl, monoalkyltriaryl-stibonium cations;
- cations formed by protonation of a base with a pK_{BH+} value of at least 20 measured in acetonitrile;
and combinations thereof, and at least one anion of a saturated and/or unsaturated (cyclo)aliphatic carbonic acid, wherein the carbonic acid is a branched carbonic acid with a length for the (cyclo)alkyl chain provided on the carboxyl group of at least 2 carbon atoms or a non-branched carbonic acid with a length for the (cyclo)alkyl chain provided on the carboxyl group of at least 4 carbon atoms.

2. A condensation cross-linking two-component dental material with a component A containing:
a) at least one polyether with an alkoxysilyl function;
and a component B containing:
b) at least one catalyst; and
c) water;
wherein the at least one catalyst b) is a salt which is formed from at least one cation selected from the group consisting of:
- complexes of alkali metal or ammonium cations with crown ethers and/or cryptands;
- tetraalkyl, tetraaryl, trialkylaryl, dialkyldiaryl, monoalkyltriaryl-ammonium cations, tetraalkyl, tetraaryl, trialkylaryl, dialkyldiaryl, monoalkyltriaryl-phosphonium cations, tetraalkyl, tetraaryl, trialkylaryl, dialkyldiaryl, monoalkyltriaryl-arsonium cations, tetraalkyl, tetraaryl, trialkylaryl, dialkyldiaryl, monoalkyltriaryl-stibonium cations;
- cations formed by protonation of a base with a pK_{BH+} value of at least 20 measured in acetonitrile; and combinations thereof, and at least one anion of a saturated and/or unsaturated (cyclo)aliphatic carbonic acid, wherein the carbonic acid is a branched carbonic acid with a length for the (cyclo)alkyl chain provided on the carboxyl group of at least 2 carbon atoms or a non-branched carbonic acid with a length for the (cyclo)alkyl chain provided on the carboxyl group of at least 4 carbon atoms.

3. The condensation cross-linking dental material according to claim 1, **characterized in that** it contains at least one reinforcing filler d₁) having a BET surface area of at least 50 m²/g and/or at least one non-reinforcing filler d₂) having a BET surface area of less than 50 m²/g.

4. The condensation cross-linking dental material according to claim 2, **characterized in that** it contains, in component A and/or in component B, at least one reinforcing filler d₁) having a BET surface area of at least 50 m²/g and/or at least one non-reinforcing filler d₂) having a BET surface area of less than 50 m²/g.

5. The dental material according to one of the preceding claims, **characterized in that** the cation of the catalyst salt b) is a complex of one or more lithium, sodium, potassium, rubidium, caesium and/or ammonium ions and one or more crown ethers and/or cryptands selected from the group consisting of: 15-crown-5, 18-crown-6, dibenzo-18-crown-6, dibenzo-21-crown-7, dibenzo-24-crown-8, dibenzo-30-crown-10, 1,4,10-trioxa-7,13-diazacyclopentadecane, 4,7,13,18-tetraoxa-1,10-diazabicyclo[8.5.5]eicosan, 1,4,10,13-tetraoxa-7,16-diazacyclooctadecane, 3,6,9,14-tetrathiabicyclo[9.2.1]tetradeca-11,13-diene, 1,4,7,10-tetrathiacyclododecane, 1,5,9,13-tetrathiacyclohexadecan-3,11-diol, 1,5,9-triazacyclododecane, 1,4,7-triazacyclononane, 1,4,7,10,13,16-hexamethyl-1,4,7,10,13,16-hexaazacyclooctadecane, 1,4,10-trioxa-7,13-diazacyclopentadecane, 4,7,13,18-tetraoxa-1,10-diazabicyclo[8.5.5]eicosan, 1,4,10,13-tetraoxa-7,16-diazacyclooctadecane, dibenzo-21-crown-7, dibenzo-24-crown-8, dibenzo-30-crown-10, 18-crown-6, 15-crown-5, 3,6,9,14-tetrathiabicyclo[9.2.1]tetradeca-11,13-diene, 1,4,7,10-tetrathiacyclododecane, 1,5,9,13-tetrathiacyclohexadecan-3,11-diol, 1,5,9-triazacyclododecane and 1,4,7-triazacyclononane.

6. The dental material according to one of claims 1 to 4, **characterized in that** the cation of the catalyst salt b) is an ion selected from the group consisting of tetramethyl-ammonium, tetraethyl-ammonium, tetrapropyl-ammonium, tetrabutyl-ammonium, tetrapentyl-ammonium, tetrahexyl-ammonium, tetraheptyl-ammonium, tetraoctyl-ammonium, tetranonylammonium, tetradecyl-ammonium, tetramethylphosphonium, tetraethyl-phosphonium, tetrapropylphosphonium, tetrabutyl-phosphonium, tetrapentylphosphonium, tetrahexyl-phosphonium, tetraheptylphosphonium, tetraoctyl-phosphonium, tetranonylphosphonium, tetradecyl-phosphonium, tetramethylarsonium, tetraethyl-arsonium, tetrapropyl-arsonium, tetrabutyl-arsonium, tetrapentyl-arsonium, tetrahexylarsonium, tetraheptyl-arsonium, tetraoctyl-arsonium, tetranonyl-arsonium, tetradecyl-arsonium, tetramethylstibonium, tetraethyl-stibonium, tetrapropylstibonium, tetrabutyl-stibonium, tetrapentylstibonium, tetrahexyl-stibonium, tetraheptylstibonium, tetraoctyl-stibonium, tetranonyl-stibonium, tetradecyl-stibonium, lauryl trimethyl-ammonium, myristyl trimethyl-ammonium, cetyl trimethyl-ammonium, stearyl trimethyl-ammonium, lauralkonium, myristalkonium, cetalkonium, stearalkonium, cetylethyldimethyl-ammonium, benzyltriethyl-ammonium, and benzalkonium cations, and combinations thereof.

7. The dental material according to one of claims 1 to 4, **characterized in that** the cation of the catalyst salt b) is an ion formed by protonation of a base with a pK_{BH+} value of at least 21 measured in acetonitrile.

8. The dental material according to claim 7, **characterized in that** the catalyst salt b) is formed from a base which has at least one structural unit in accordance with general formula I: 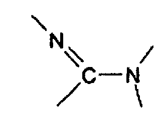 and/or in accordance with general formula II: 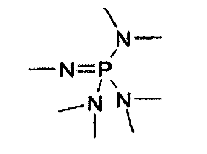 and/or in accordance with general formula III:

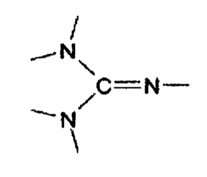

9. The dental material according to claim 7 or claim 8, **characterized in that** the cation of the catalyst salt b) is a protonated base selected from the group consisting of 1,1,3,3-tetramethylguanidine, diazabicylo[5.4.0]undec-7-ene, 1,5-diazabicyclo[4.3.0]non-5-ene, tert-butylimino-tris(dimethylamino)phosphorane, tert-butyliminotri(pyrrolidino)-phosphorane, tert-octylimino-tris(dimethylamino)phosphorane, 2-tert-butylimino-2-diethylamino-1,3-dimethyl-perhydro-1,3,2-diazaphosphorin, 2-tert-butylimino-2-diethylamino-1,3-dimethyl-perhydro-1,3,2-diazaphosphorin on polystyrene, 1-tert-butyl-2,2,4,4,4-pentakis(diethylamino)-2Λ5, 4Λ5-catenadi(phosphazene), 1-ethyl-2,2,4,4,4-pentakis(diethylamino)-2Λ5, 4Λ5-catenadi(phosphazene), 1-tert-butyl-4,4,4-tris(dimethylamino)-2,2-bis[tris(dimethylamino)-phosphoranylidenamino]-2Λ⁵, 4Λ⁵-catenadi(phosphazene), 1-tert-octyl-4,4,4-tris(dimethylamino)-2,2-bis[tris(dimethylamino)-phosphoranylidenamino]-2Λ⁵, 4Λ⁵-catenadi(phosphazene), 2,8,9-triisobutyl-2,5,8,9-tetraaza-1-phosphabicyclo[3.3.3]undecane, 2,8,9-triisopropyl-2,5,8,9-tetraaza-1-phospha-bicyclo[3.3.3]undecane, 2,8,9-trimethyl-2,5,8,9-tetraaza-1-phosphabicyclo[3.3.3]undecane, 1,8-bis(tetramethylguanidino)naphthalene, 2-tert-butyl-1,1,3,3-tetramethylguanidine, 1,5,7-triazabicyclo(4.4.0)dec-5-ene, 7-methyl-1,5,7-triazabicyclo(4.4.0)dec-5-ene, 1,5-diazabicyclo[4.3.0)non-5-ene and 3,3,6,9,9-pentamethyl-2,10-diazabicyclo-(4.4.0)dec-1-ene, preferably a protonated base selected from the group consisting of tert-butyliminotri(pyrrolidino)-phosphorane, 1-tert-butyl-2,2,4,4,4-pentakis(diethylamino)-2Λ5, 4Λ5-catenadi(phosphazene), 1-tert-butyl-4,4,4-tris(dimethylamino)-2,2-bis[tris(dimethylamino)-phosphoranylidenamino]-2Λ⁵, 4Λ⁵-catenadi(phosphazene), tert-octylimino-tris(dimethylamino)phosphorane, 2,8,9-triisopropyl-2,5,8,9-tetraaza-1-phosphabicyclo[3.3.3]undecane, 1,5-diazabicyclo[4.3.0]non-5-ene, 1,1,3,3-tetramethylguanidine, diazabicyclo[5.4.0]undec-7-ene, 7-methyl-1,5,7-triazabicyclo(4.4.0)dec-5-ene, 2-tert-butyl-1,1,3,3-tetramethylguanidine, 1,5,7-triazabicyclo(4.4.0)dec-5-ene and/or 1,8-bis(tetramethylguanidino)naphthalene.

10. The dental material according to one of the preceding claims, **characterized in that** the anion of the catalyst salt b) is an anion of a branched carbonic acid with a length for the (cyclo)alkyl chain provided on the carboxyl group of at least 3 carbon atoms or a non-branched carbonic acid with a length for the (cyclo)alkyl chain provided on the carboxyl group of at least 5 carbon atoms.

11. The dental material according to one of the preceding claims, **characterized in that** the anion of the catalyst salt b) is a deprotonated, saturated and/or unsaturated (cyclo)aliphatic carbonic acid wherein the (cyclo)alkyl chain which is in the position α to the carboxyl group has at least one branch.

12. The dental material according to one of the preceding claims, **characterized in that** the anion of the catalyst salt b) is an ion selected from the group consisting of deprotonated 2,2-dialkylalkanoic acids, 3,3-dialkylalkanoic acids, 4,4-dialkylalkanoic acids, 2,3-dialkylalkanoic acids, 2,4-dialkylalkanoic acids, 3,4-dialkylalkanoic acids, 2,2-dialkylalkenoic acids, 3,3-dialkylalkenoic acids, 4,4-dialkylalkenoic acids, 2,3-dialkylalkenoic acids, 2,4-dialkylalkenoic acids, 3,4-dialkylalkenoic acids, 2,2-dialkylalkynoic acids, 3,3-dialkylalkynoic acids, 4,4-dialkylalkynoic acids, 2,3-dialkylalkynoic acids, 2,4-dialkylalkynoic acids, 3,4-dialkylalkynoic acids, 2-monoalkylalkanoic acids, 3-monoalkylalkanoic acids, 4-monoalkylalkanoic acids, 2,2-dialkylhexanoic acids, preferably 2,2-dialkylnonanoic acid, 2,2-dimethyl-decanoic acid, 2,2-diethyldecanoic acid, 2,2-dipropyldecanoic acid, 2,2-dibutyldecanoic acid, 2,2-dimethylnonanoic acid, 2,2-diethylnonanoic acid, 2,2-dipropylnonanoic acid, 2,2-dibutylnonanoic acid, 2,2-dimethyloctanoic acid, 2,2-diethyloctanoic acid, 2,2-dipropyloctanoic acid, 2,2-dibutyloctanoic acid, 2,2-dimethylheptanoic acid, 2,2-diethylheptanoic acid, 2,2-dipropylheptanoic acid, 2,2-dibutylheptanoic acid, 2,2-dimethylhexanoic acid, 2,2-diethylhexanoic acid, 2,2-dipropylhexanoic acid, 2,2-dibutylhexanoic acid, 2-butyloctanoic acid, 2-hexyldecanoic acid, 2-propylpentanoic acid, 1-methyl-1-cyclohexanecarbonic acid, 2,2-dimethylbutanoic acid, 2,2-dimethylvalerianic acid, 3,5,5-trimethylhexanoic acid, 2-ethylhexanoic acid, decanoic acid, octanoic acid, hexanoic acid and enanthic acid.

13. The dental material according to one of the preceding claims, **characterized in that** it contains 0.001 to 1 mmol/g of at least one catalyst b) with respect to the total mixture.

14. The dental material according to one of the preceding claims, **characterized in that** the catalyst salt used in the polyether matrix is of sufficiently high solubility to result, when used in an amount of 0.001 to 1 mmol/g with respect to the total mixture, in curing of the dental material, determined by recovery after deformation in accordance with ISO 4823 (version 1992) in 30 minutes or less for a dental impression composition.

15. The dental material according to claim 2, **characterized in that** it contains as catalyst b) at least one salt of 1,8-diazabicyclo[5.4.0]undec-7-ene, 1,5-diazabicyclo[4.3.0]non-5-ene and/or 1,1,3,3-tetramethylguanidine with 2-ethylhexanoic acid.

16. The dental material according to one of the preceding claims, **characterized in that** apart from one or more salts in accordance with one of claims 1 to 15, it contains no other catalyst, in particular no organometallic compounds, no heavy metal carboxylate salts, tertiary amines or free acids.

17. A dental material according to one of the preceding claims, **characterized in that** the at least one polyether with an alkoxysilyl function a) has, as a third structural unit, alkylene spacers on each of the terminal alkoxysilyl groups which, particularly preferably, are C₁-C₆ alkyl groups, and has, as the fourth structural unit, 0 to 8 mmol/g of urethane groups and/or 0 to 8 mmol/g of urea groups.

18. The dental material according to claim 17, **characterized in that** the at least one polyether with an alkoxysilyl function a) contains no urethane and/or urea groups within the polymer chain and at most one or no more than one urethane and/or urea group and at most one or no more than one methylene spacer group on each chain end.

19. The dental material according to claim 17, **characterized in that** the individual structural units of the at least one polyether a) are as follows: 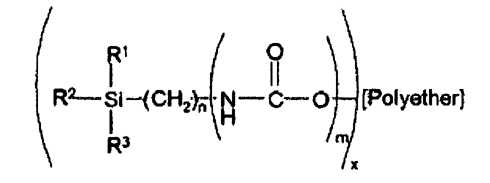 wherein R¹, R² and R³ independently of each other is/are alkoxy, alkyl, aryl, aralkyl, alkylaryl groups or hydrogen, preferably butoxy, propoxy, ethoxy, methoxy, hexyl, pentyl, butyl, propyl, ethyl or methyl groups, particularly preferably ethoxy, methoxy, ethyl or methyl groups, provided that at least one of said residues, preferably two or all three residues, are alkoxy groups; and wherein
x = 1 to 6, preferably x = 2 to 4 and particularly preferably x = 2;
n = 1 to 6, preferably n = 1 to 3 and particularly preferably n = 1; and wherein m = 0 or 1, particularly preferably m = 1;
and/or 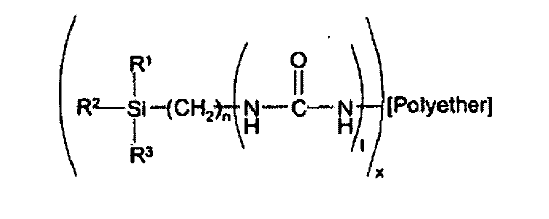 wherein R¹, R² and R³ independently of each other is/are alkoxy, alkyl, aryl, aralkyl, alkylaryl groups or hydrogen, preferably butoxy, propoxy, ethoxy, methoxy, hexyl, pentyl, butyl, propyl, ethyl or methyl groups, particularly preferably ethoxy, methoxy, ethyl or methyl groups, provided that at least one of said residues, preferably two or all three residues, are alkoxy groups; and wherein
x = 1 to 6, preferably x = 2 to 4 and particularly preferably x = 2;
n = 1 to 6, preferably n = 1 to 3 and particularly preferably n = 1; and wherein l = 0 or 1, particularly preferably l = 1.

20. The dental material according to claim 19, **characterized in that** n equals 1.

21. The dental material according to one of the preceding claims, **characterized in that** it contains at least one water scavenger g), preferably a water scavenger selected from the group consisting of alkoxysilanes, titanates, zirconates, zeolites, aluminium sulphate, anhydrous calcium sulphate, Blue Gel, oxazolidines, alkoxysilanes, and particularly preferably a water scavenger selected from the group consisting of vinyltrimethoxysilane, N-trimethoxysilylmethyl-O-methylcarbamate, and 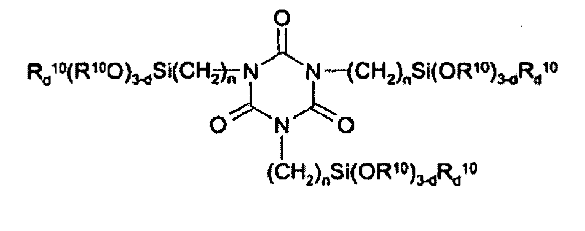 wherein n = 1 to 6, preferably n = 1 or 3, particularly preferably n = 1, d = 0 or 1; and
R¹⁰ is a linear or branched C₁-C₃₀ alkyl residue, in which the hydrogen atoms may be partially substituted with halogen atoms, OH-, NH₂-, NO₂- or other C₁-C₆ alkyl residues.

22. The dental material according to one of the preceding claims, **characterized in that** it contains at least one paste former h), preferably a paste former selected from the group consisting of polyethers, polyvinylpyrrolidones, polyurethanes, polyesters, waxes, Vaseline, paraffin oils, silicone oils, polyfunctional alcohols, propylene glycols, polypropylene glycols, ethylene glycols, polyethylene glycols, copolymerisates formed from N-vinylpyrrolidone and vinyl acetate, carboxymethyl, methyl, hydroxyethyl or hydroxypropyl cellulose, polysaccharides, glycerin and poly(meth)acrylic acids.

23. A mixture obtainable by mixing components A and B of the two-component dental material according to one of claims 2 to 22, **characterized in that** the base component A is mixed with the catalyst component B in a ratio of 1:1 to 20:1.

24. Use of a dental material according to one of claims 1 to 23 in dentistry and/or dental technology.

## Revendications

1. Matériau dentaire à réticulation par condensation, contenant :
a) au moins un polyéther aux fonctions alkoxysilyles et
b) au moins un catalyseur,
**caractérisé en ce que** l'au moins un catalyseur b) est un sel formé à partir d'au moins un cation choisi dans le groupe constitué par
- les complexes de cations de métaux alcalins ou d'ammonium avec les éthers couronnes et/ou les cryptands,
- les cations de tétraalkyl-, tétraaryl-, trialkylaryl-, dialkyldiaryl-, monoalkyltriarylammonium, les cations de tétraalkyl-, tétraaryl-, trialkylaryl-, dialkyldiaryl-, monoalkyltriarylphosphonium, les cations de tétraalkyl-, tétraaryl-, trialkylaryl-, dialkyldiaryl-, monoalkyltriarylarsonium, les cations de tétraalkyl-, tétraaryl-, trialkylaryl-, dialkyldiaryl-, monoalkyltriarylstibonium,
- les cations formés par protonation d'une base dont la valeur pK_{BH+}, mesurée dans de l'acétonitrile, est d'au moins 20
et par leurs combinaisons, et à partir d'au moins un anion d'un acide carboxylique (cyclo)aliphatique saturé et/ou insaturé, ledit acide carboxylique étant soit un acide carboxylique ramifié dont le groupe carboxyle est pourvu d'une chaîne (cyclo)alkyle d'une longueur d'au moins 2 atomes de carbone soit un acide carboxylique linéaire dont le groupe carboxyle est pourvu d'une chaîne (cyclo)alkyle d'une longueur d'au moins 4 atomes de carbone.

2. Matériau dentaire à réticulation par condensation, constitué de deux composants dont un composant A contient
a) au moins un polyéther aux fonctions alkoxysilyles et dont un composant B contient
b) au moins un catalyseur et
c) de l'eau,
l'au moins au catalyseur b) étant un sel formé à partir d'au moins un cation choisi dans le groupe constitué par
- les complexes de cations de métaux alcalins ou d'ammonium avec les éthers couronnes et/ou les cryptands,
- les cations de tétraalkyl-, tétraaryl-, trialkylaryl-, dialkyldiaryl-, monoalkyltriarylammonium, les cations de tétraalkyl-, tétraaryl-, trialkylaryl-, dialkyldiaryl-, monoalkyltriarylphosphonium, les cations de tétraalkyl-, tétraaryl-, trialkylaryl-, dialkyldiaryl-, monoalkyltriarylarsonium, les cations de tétraalkyl-, tétraaryl-, trialkylaryl-, dialkyldiaryl-, monoalkyltriarylstibonium,
- les cations formés par protonation d'une base dont la valeur pK_{BH+}, mesurée dans de l'acétonitrile, est d'au moins 20 et par leurs combinaisons, et à partir d'au moins un anion d'un acide carboxylique (cyclo)aliphatique saturé et/ou insaturé, ledit acide carboxylique étant soit un acide carboxylique ramifié dont le groupe carboxyle est pourvu d'une chaîne (cyclo)alkyle d'une longueur d'au moins 2 atomes de carbone soit un acide carboxylique linéaire dont le groupe carboxyle est pourvu d'une chaîne (cyclo)alkyle d'une longueur d'au moins 4 atomes de carbone.

3. Matériau dentaire à réticulation par condensation selon la revendication 1, **caractérisé en ce qu'**il contient au moins une charge renforçante d₁) dont la surface BET est d'au moins 50 m²/g et/ou au moins une non-renforçante d₂) dont la surface BET est d'au moins 50 m²/g_{.}

4. Matériau dentaire à réticulation par condensation et constitué de deux composants selon la revendication 2, **caractérisé en ce qu'**il contient, dans le composant A et/ou dans le composant B au moins une charge renforçante d₁) dont la surface BET est d'au moins 50 m²/g et/ou au moins une non-renforçante d₂) dont la surface BET est d'au moins 50 m²/g.

5. Matériau dentaire selon l'une des revendications précédentes, **caractérisé en ce que** le cation du sel catalyseur b) est un complexe constitué d'un ou de plusieurs ions de lithium, de sodium, de potassium, de rubidium, de césium et/ou d'ammonium et d'un ou de plusieurs éther couronnes et/ou cryptands choisis dans le groupe constitué par le 15-couronne-5, le 18-couronne-6, le dibenzo-18-couronne-6, le dibenzo-21-couronne-7, le dibenzo-24-couronne-8, le dibenzo-30-couronne-10, le 1,4,10-trioxa-7,13-diazacyclopentadécane, le 4,7,13,18-tétraoxa-1,10-diazabicyclo[8.5.5]eicosane, le 1,4,10,13-tétraoxa-7,16-diazacyclooctadécane, le 3,6,9,14-tétrathiabicyclo[9.2.1)tétradéca-11,13-diène, le 1,4,7,10-tétrathiacyclododécane, le 1,5,9,13-tétrathiacyclohexadécane-3,11-diol, le 1,5,9-Triazacyclododécane, le 1,4,7-Triazacyclononane, le 1,4,7,10,13,16-hexamethyl-1,4,7,10,13,16-hexaazacyclooctadécane, le 1,4,10-trioxa-7,13-diazacyclopentadécane, le 4,7,13,18-tétraoxa-1,10-diazabicyclo[8.5.5]eicosane, le 1,4,10,13-tétraoxa-7,16-diazacyclooctadécane, le dibenzo-21-couronne-7, le dibenzo-24-couronne-8, le dibenzo-30-couronne-10, le 18-couronne-6, le 15-couronne-5, le 3,6,9,14-tétrathiabicyclo[9.2.1]tétradeca-11,13-diène, le 1,4,7,10-tétrathiacyclododécane, le 1,5,9,13-tétrathiacyclohexadécane-3,11-diol, le 1,5,9-triazacyclododécane et le 1,4,7-triazacyclononane.

6. Matériau dentaire selon l'une des revendications 1 à 4, **caractérisé en ce que** le cation du sel catalyseur b) est un ion choisi dans le groupe constitué par les cations de tétraméthylammonium, de tétraéthylammonium, de tétrapropylammonium, de tétrabutylammonium, de tétrapentylammonium, de tétrahexylammonium, de tétraheptylammonium, de tétraoctylammonium, de tétranonylammonium, de tétradecylammonium, de tétraméthylphosphonium, de tétraéthylphosphonium, de tétrapropylphosphonium, de tétrabutylphosphonium, de tétrapentylphosphonium, de tétrahexylphosphonium, de tétraheptylphosphonium, de tétraoctylphosphonium, de tétranonylphosphonium, de tétradecylphosphonium, de tétraméthylarsonium, de tétraéthylarsonium, de tétrapropylarsonium, de tétrabutylarsonium, de tétrapentylarsonium, de tétrahexylarsonium, de tétraheptylarsonium, de tétraoctylarsonium, de tétranonylarsonium, de tétradecylarsonium, de tétraméthylstibonium, de tétraéthylstibonium, de tétrapropylstibonium, de tétrabutylstibonium, de tétrapentylstibonium, de tétrahexylstibonium, de tétraheptylstibonium, de tétraoctylstibonium, de tétranonylstibonlum, de tétradecylstibonium, de lauryltriméthylammonium, de myristyltriméthylammonium, de cétyltriméthylammonium, de stéaryltriméthylammonium, de lauralkonium, de myristalkonium, de cétalkonium, de stéaralkonium, de cétyléthyldiméthylammonium, de benzyltriéthylammonium, de benzalkonium et de leurs combinaisons.

7. Matériau dentaire selon l'une des revendications 1 à 4, **caractérisé en ce que** le cation du sel catalyseur b) est un ion formé par protonation d'une base dont la valeur pK_{BH+}, mesurée dans de l'acétonitrile, est d'au moins 21.

8. Matériau dentaire selon la revendication 7, **caractérisé en ce que** le sel catalyseur b) formé à partir d'une base comportant au moins une unité structurelle selon la formule générale I 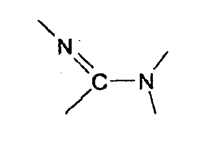 et/ou selon la formule générale II 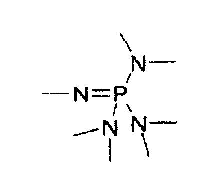 et/ou selon la formule générale III

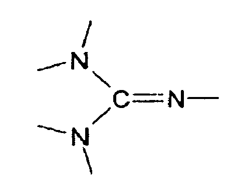

9. Matériau dentaire selon les revendications 7 ou 8, **caractérisé en ce qu'**on utilise en tant que cation du sel catalyseur b) une base protonée choisié dans le groupe constitué par la 1,1,3,3-tétraméthylguanidine, le diazabicyclo[5.4.0]undéc-7-ène, le 1,5-diazabicyclo[4.3.0]non-5-ène, le tert-butylimino-tris(diméthylamino)phosphorane, le tert-butyliminotri(pyrrolidino)-phosphorane, le tert-octylimino-tris(diméthylamino)phosphorane, la 2-tert-butylimino-2-diéthylamino-1,3-diméthyl-perhydro-1,3,2-diazaphosphorine, la 2-tert-butylimino-2-diéthylamino-1,3-diméthyl-perhydro-1,3,2-diazaphosphorine sur polystyrène, le 1-tert-butyl-2,2,4,4,4-pentakis(diéthylamino)-2Λ5, le 4Λ5-catenadi(phosphazène), le 1-éthyl-2,2,4,4,4-pentakis(diéthylamino)-2Λ5, le 4Λ5-catenadi(phosphazène), le 1-tert-butyl-4,4,4-tris(diméthylamino)-2,2-bis[tris(diméthylamino)-phosphoraneylidèneamino]-2Λ⁵, le 4Λ⁵-catenadi(phosphazène), le 1-tert-octyl-4,4,4-tris(diméthylamino)-2,2-bis[tris(diméthylamino)-phosphoraneylidèneamino]2Λ⁵, le 4Λ⁵-catenadi(phosphazène), le 2,8,9-triisobutyl-2,5,8,9-tétraaza-1-phosphabicyclo[3.3.3]undécane, le 2,8,9-triisopropyl-2,5,8,9-tétraaza-1-phosphabicyclo[3.3.3]undécane, le 2,8,9-triméthyl-2,5,8,9-tétraaza-1-phosphabicyclo[3.3.3]undécane, le 1,8-bis(tétraméthylguanidino)naphthalène, la 2-tert-butyl-1,1,3,3-tétraméthylguanidine, le 1,5,7-triazabicyclo(4.4.0)dec-5-ène, le 7-méthyl-1,5,7-triazabicyclo(4.4.0)dec-5-ène, le 1,5-diazabicyclo[4.3.0)non-5-ène et le 3,3,6,9,9-pentaméthyl-2,10-diazabicyclo-(4.4.0)dec-1-ène, s'agissant de préférence d'une base protonée choisie dans le groupe constitué par le tert-butyliminotri(pyrrolidino)phosphorane, le 1-tert-butyl-2,2,4,4,4-pentakis(diéthylamino)-2Λ5, le 4Λ5-catenadi(phosphazène), le 1-tert-butyl-4,4,4-tris(diméthylamino)-2,2-bis[tris(diméthylamino)-phosphoraneylidèneamino]-2Λ⁵, le 4Λ⁵-catenadi(phosphazène), le tert-octylimino-tris(diméthylamino)phosphorane, le 2,8,9-tri-isopropyl-2,5,8,9-tétraaza-1-phosphabicyclo[3.3.3]undécane, le 1,5-diazabicyclo[4.3.0]non-5-ène, la 1,1,3,3-tétraméthylguanidine, le diazabicyclo[5.4.0]undéc-7-ène, le 7-méthyl-1,5,7-triazabicyclo(4.4.0)dec-5-ène, la 2-tert-butyl-1,1,3,3-tétra-méthylguanidine, le 1,5,7-triazabicyclo(4.4.0)dec-5-ène et/ou le 1,8-bis(tétraméthylguanidino)naphthalène.

10. Matériau dentaire selon l'une des revendications précédentes, **caractérisé en ce que** l'anion du sel catalyseur b) est un anion soit d'un acide carboxylique ramifié dont le groupe carboxyle est pourvu d'une chaîne (cyclo)alkyle d'une longueur d'au moins 3 atomes de carbone soit d'un acide carboxylique linéaire dont le groupe carboxyle est pourvu d'une chaîne (cyclo)alkyle d'une longueur d'au moins 5 atomes de carbone.

11. Matériau dentaire selon l'une des revendications précédentes, **caractérisé en ce que** l'anion du sel catalyseur b) est un acide carboxylique (cyclo)aliphatique déprotoné saturé et/ou insaturé dont la chaîne (cyclo)alkyle présente au moins une ramification située en position α par rapport au groupe carboxyle.

12. Matériau dentaire selon l'une des revendications précédentes, **caractérisé en ce que** l'anion du sel catalyseur b) est un ion choisi dans le groupe constitué par les acides 2,2-dialkylalcanoïques, les acides 3,3-dialkylalcanoïques, les acides 4,4-dialkylalcanoïques, les acides 2,3-dialkylalcanoïques, les acides 2,4-dialkylalcanoïques, les acides 3,4-dialkylalcanoïques, les acides 2,2-dialkylalcénoïques, les acides 3,3-dialkylalcénoïques, les acides 4,4-dialkylalcénoïques, les acides 2,3-dialkylalcénoïques, les acides 2,4-dialkylalcénoïques, les acides 3,4-dialkylalcénoïques, les acides 2,2-dialkylalcynoïques, les acides 3,3-dialkylalcynoïques, les acides 4,4-dialkylalcynoïques, les acides 2,3-dialkylalcynoïques, les acides 2,4-dialkylalcynoïques, les acides 3,4-dialkylalcynoïques, les acides 2-monoalkylalcanoïques, les acides 3-monoalkylalcanoïques, les acides 4-monoalkylalcanoïques, les acides 2,2-dialkylhexanoïques, s'agissant de préférence de l'acide 2,2-dialkylnonanoïque, l'acide 2,2-diméthyldecanoïque, l'acide 2,2-diéthyldecanoïque, l'acide 2,2-dipropyldecan-saure, l'acide 2,2-dibutyldecanoïque, l'acide 2,2-diméthylnonanoïque, l'acide 2,2-diéthylnonanoïque, l'acide 2,2-dipropylnonanoïque, l'acide 2,2-dibutylnonanoïque, l'acide 2,2-diméthyloctanoïque, l'acide 2,2-diéthyloctanoïque, l'acide 2,2-dipropyloctanoïque, l'acide 2,2-dibutyloctanoïque, l'acide 2,2-diméthylheptanoïque, l'acide 2,2-diéthylheptanoïque, l'acide 2,2-dipropylheptanoïque, l'acide 2,2-dibutylheptanoïque, l'acide 2,2-diméthylhexanoïque, l'acide 2,2-diéthylhexanoïque, l'acide 2,2-dipropylhexanoïque, l'acide 2,2-dibutylhexanoïque, l'acide 2-butyloctanoïque, l'acide 2-hexyldecanoïque, l'acide 2-propylpentanoïque, l'acide 1-méthyl-1-cyclohexanoïque, l'acide 2,2-diméthylbutanoïque, l'acide 2,2-diméthylpentanoïque, 3,5,5-triméthylhexanoïque, l'acide 2-éthylhexanoïque, l'acide décanoïque, l'acide octanoïque, l'acide hexanoïque et de l'acide heptanoïque.

13. Matériau dentaire selon l'une des revendications précédentes, **caractérisé en ce qu'**il contient 0,001 à 1 mmol/g, par à la totalité du mélange, de l'au moins un catalyseur b).

14. Matériau dentaire selon l'une des revendications précédentes, **caractérisé en ce que** le sel catalyseur mis en oeuvre est suffisamment soluble dans la matrice de polyéther pour effectuer le durcissement du matériau dentaire dans un délai inférieur ou égal à 30 minutes lorsqu'il est mis en oeuvre dans une quantité de 0,001 à 1 mmol/g par rapport à la totalité du mélange, le durcissement étant évalué par essai de mémoire élastique après déformation selon ISO 4823 (édition 1992).

15. Matériau dentaire selon la revendication 2, **caractérisé en ce qu'**il contient en tant que catalyseur b) au moins un sel du 1,8-diazabicyclo[5.4.0]undéc-7-ène, du 1,5-diazabicyclo[4.3.0]non-5-ène et/ou de la 1,1,3,3-tétraméthylguanidine avec l'acide 2-éthylhexanoïque.

16. Matériau dentaire selon l'une des revendications précédentes, **caractérisé en ce qu'**il contient, outre le ou les sel(s) selon l'une des revendications 1 à 15, pas d'autres catalyseurs, notamment pas de composés organométalliques, pas de sels de type carboxylates de métaux lourds, pas d'amines tertiaires et pas d'acides libres.

17. Matériau dentaire selon l'une des revendications précédentes, **caractérisé en ce que** l'au moins un polyéther aux fonctions alkoxysilyles a) présente en tant que troisième unité structurelle, sur chacun des groupes alkoxysilyles terminaux, des unités d'espacement de type alkylène, s'agissant avec une préférence particulière de groupes alkyles C₁-C₆, et en tant que quatrième unité structurelle 0 à 8 mmol/g de groupes uréthane et/ou 0 à 8 mmol/g de groupes urée.

18. Matériau dentaire selon la revendication 17, **caractérisé en ce que** l'au moins un polyéther aux fonctions alkoxysilyles a) ne contient pas de groupes uréthane et/ou de groupes urée au sein de sa chaîne polymère et qu'il porte, à chaque extrémité de sa chaîne, au plus un ou, autrement dit, pas plus d'un groupe de type uréthane et/ou urée ainsi qu'au plus un ou, autrement dit, pas plus d'un groupe d'espacement de type méthylène.

19. Matériau dentaire selon la revendication 17, **caractérisé en ce que** chacune des unités structurelles de l'au moins un polyéther a) est disposée selon 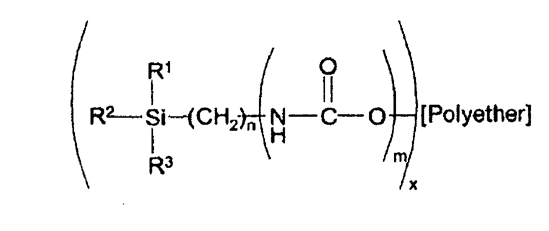 R¹, R² et R³ étant, indépendamment l'un de l'autre, des groupes alkoxy, alkyle, aryle, aralkyle, alkylaryle ou de l'hydrogène, s'agissant de préférence de groupes butoxy, propoxy, éthoxy, méthoxy, hexyle, pentyle, butyle, propyle, éthyle ou méthyle et avec une préférence particulière de groupes éthoxy, méthoxy, éthyle ou méthyle, à condition qu'au au moins un et préférentiellement deux ou tous les trois des radicaux précités soient des groupes alkoxy, et
x = 1 à 6, de préférence x = 2 à 4 et, avec une préférence particulière, x = 2, n = 1 à 6, de préférence n = 1 à 3 et, avec une préférence particulière, n = 1 ainsi que m = 0 ou 1, avec une préférence particulière m = 1,
et/ou 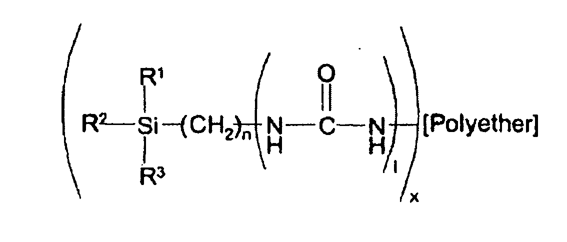 R¹, R² et R³ étant, indépendamment l'un de l'autre, des groupes alkoxy, alkyle, aryle, aralkyle, alkylaryle ou de l'hydrogène, s'agissant de préférence de groupes butoxy, propoxy, éthoxy, méthoxy, hexyle, pentyle, butyle, propyle, éthyle ou méthyle et avec une préférence particulière de groupes éthoxy, méthoxy, éthyle ou méthyle, à condition qu'au au moins un et préférentiellement deux ou tous les trois des radicaux précités soient des groupes alkoxy, et
x = 1 à 6, de préférence x = 2 à 4 et, avec une préférence particulière, x = 2, n = 1 à 6, de préférence n = 1 à 3 et, avec une préférence particulière, n = 1 ainsi que l = 0 ou 1, avec une préférence particulière l = 1.

20. Matériau dentaire selon la revendication 19, **caractérisé en ce que** n égale 1.

21. Matériau dentaire selon l'une des revendications précédentes, **caractérisé en ce qu'i**l contient au moins un absorbant d'eau g), s'agissant d'un absorbant d'eau choisi de préférence dans le groupe constitué par les alkoxysilanes, les titanates, les zirconates, les zéolithes, le sulfate d'aluminium, le sulfate de calcium anhydre, le gel de silice bleu, les oxazolidines et les alkoxysilanes et, avec une préférence particulière, dans le groupe constitué de vinyltriméthylsilane, de N-trimethoxysilylméthyl-O-méthylcarbamate et de 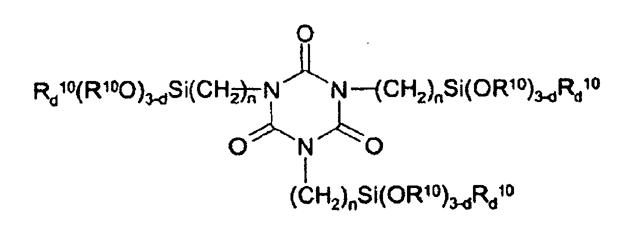 dans lequel n = 1 à 6, de préférence n = 1 ou 3 et, avec une préférence particulière, n = 1, d = 0 ou 1, et
R¹⁰ = un radical C₁-C₃₀-alkyle linéaire ou ramifié dans lequel les atomes d'hydrogène peuvent partiellement être substitués par des atomes d'halogènes, par OH-, NH₂-, NO₂- ou par d'autres radicaux C₁-C₆-alkyle.

22. Matériau dentaire selon l'une des revendications précédentes, **caractérisé en ce qu'**il contient au moins une matrice pour préparations pâteuses h), s'agissant de préférence d'une matrice pour préparations pâteuses choisie dans le groupe constitué par les polyéthers, les polyvinylpyrrolidones, polyuréthanes, les polyesters, les cires, la vaseline, les paraffines liquides, les huiles de silicone, les polyols, les propylèneglycols, les polypropylèneglycols, les éthylèneglycols, les polyéthylèneglycols, les copolymérisats de N-vinylpyrrolidone et d'acétate de vinyle, les carboxyméthyl-, méthyl-, hydroxyéthyl-, hydroxypropyl-celluloses, les polysaccharides, le glycérol et les acides poly(méth)acryliques.

23. Mélange obtenu en mélangeant les composants A et B du matériau dentaire à deux composants selon l'une des revendications 2 à 22, **caractérisé en ce que** le composant de base A est mélangé avec le composant catalyseur B dans le rapport compris entre 1:1 et 20:1.

24. Utilisation d'un matériau dentaire selon l'une des revendications 1 à 23 dans la médecine dentaire et/ou la technique dentaire.
